(19) Europäisches Patentamt
European Patent Office
Office européen des brevets

(11) Veröffentlichungsnummer: **0 008 058**
**B1**

(12) # EUROPÄISCHE PATENTSCHRIFT

(45) Veröffentlichungstag der Patentschrift: **18.08.82**

(21) Anmeldenummer: **79102707.1**

(22) Anmeldetag: **30.07.79**

(51) Int. Cl.³: **C 07 D 211/98,** C 07 D 401/12, C 07 D 405/12, C 07 D 409/12, A 61 K 31/445, A 23 K 1/00

(54) **N-Amino-3,4,5-trihydroxypiperidine, Verfahren zu ihrer Herstellung, sie enthaltende Arzneimittel, Verfahren zu deren Herstellung und die genannten Verbindungen enthaltende Tierfuttermittel.**

(30) Priorität: **10.08.78 DE 2835069**

(43) Veröffentlichungstag der Anmeldung: **20.02.80 Patentblatt 80/4**

(45) Bekanntmachung des Hinweises auf die Patenterteilung: **18.08.82 Patentblatt 82/33**

(84) Benannte Vertragsstaaten: **AT BE CH DE FR GB IT NL**

(56) Entgegenhaltungen:
**DE A 2 656 602**
**DE A 2 658 561**
**US A 3 927 001**

**Die Akte enthält technische Angaben, die nach dem Eingang der Anmeldung eingereicht wurden und die nicht in dieser Patentschrift enthalten sind.**

(73) Patentinhaber: **BAYER AG, Zentralbereich Patente, Marken und Lizenzen, D-5090 Leverkusen 1, Bayerwerk (DE)**

(72) Erfinder: **Kinast, Günther, Dr., Am Eckbusch 42, D-5600 Wuppertal 1 (DE)**
Erfinder: **Müller, Lutz, Dr., Kronprinzenallee 111, D-5600 Wuppertal 1 (DE)**
Erfinder: **Puls, Walter, Dr., In den Birken 75, D-5600 Wuppertal 1 (DE)**
Erfinder: **Sitt, Rüdiger, Dr., Claudiusweg 9, D-5600 Wuppertal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 008 058 B1

# N-Amino-3,4,5-trihydroxypiperidine, Verfahren zu ihrer Herstellung, sie enthaltende Arzneimittel, Verfahren zu deren Herstellung und die genannten Verbindungen enthaltende Tierfuttermittel

Die vorliegende Erfindung betrifft N-Amino-3,4,5-trihydroxypiperidine, Verfahren zu ihrer Herstellung sowie ihre Verwendung als Arzneimittel, insbesondere als Mittel gegen Diabetes, Hyperlipoproteinämie, Adipositas, Meteorismus, Flatulenz, gastrointestinale Infektionen, Hypertension, Karies und Atherosklerose sowie in der Tierernährung zur Beeinflussung des Fleisch/Fett-Verhältnisses zugunsten des Fleischanteils.

Die am Stickstoffatom unsubstituierte Verbindung und sie enthaltende antidiabetische Mittel sind bereits z.B. aus der DE-A 26 56602 bekannt. Auch die DE-A-26 58561 beschreibt die Verwendung von u.a. Nojirimycin und Desoxynojirimycin als Inhibitoren für Glucosidasen des Verdauungstraktes.

Die erfindungsgemässen Verbindungen entsprechen der Formel (I),

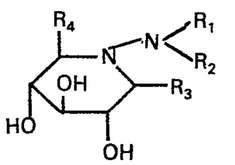

(I)

worin

$R_1$ Wasserstoff, $R_5$, Formyl, $COR_5$, $CO_2R_5$, Carbonamid, $CONHR_5$, $CONR_5R_6$, $CSR_5$, $CSNH_2$, $CSNHR_5$, $CSNR_5R_6$, $SO_3H$ oder $SO_2R_5$,

$R_2$ Wasserstoff oder $R_5$ oder $R_1$ und $R_2$ gemeinsam die Gruppierung

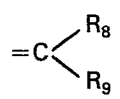

$R_3$ Wasserstoff, Hydroxy, $OR_5$, Mercapto, $SR_5$, Amino, $NHR_5$, $NR_5R_6$, Cyano, Carboxy, $CO_2R_5$, Carbonamido, $CONHR_5$, $CONR_5R_6$, Aminomethyl, $CH_2NHR_5$, $CH_2NR_5R_6$, $CH_2-NR_5COR_6$, $CH_2NHSO_2R_5$, $CH_2NR_5SO_2R_6$, Sulfo, Hydroxymethyl, $CH_2OR_5$, oder $CH_2\ OCOR_5$,

$R_4$ Hydroxymethyl, Hydroxy-($C_1$–$C_6$-Alkyl)-methyl, $C_1$–$C_7$-Alkyl und $C_1$–$C_5$-Alkoxy-methyl,

$R_5$, $R_6$ und $R_7$ unabhängig voneinander einen gegebenenfalls substituierten, geradkettigen, verzweigten oder cyclischen gesättigten oder ungesättigten aliphatischen Kohlenwasserstoffrest oder einen gegebenenfalls substituierten aromatischen oder heterocyclischen Rest, wobei 2 Reste $R_5$, $R_6$ und $R_7$ auch miteinander verknüpft sein können,

$R_8$ und $R_9$ unabhängig voneinander Wasserstoff oder $R_5$ bedeuten.

Bevorzugt bedeuten $R_5$, $R_6$ und $R_7$ Alkyl mit 1 bis 30, insbesondere 1 bis 18 C-Atome, Alkenyl oder Alkinyl mit 2 bis 18, insbesondere 3 bis 10 C-Atomen, Mono-, Bi- oder Tricycloalkyl, -alkenyl oder -akadienyl mit 3 bis 10 C-Atomen, Aryl mit 6 oder 10 C-Atomen, einen heterocyclischen Rest mit 3 bis 8, insbesondere 3 bis 6 Ringgliedern, der 1, 2, 3 oder 4 Heteroatome, insbesondere N, O oder S enthalten kann und an dem ein Benzolring oder ein weiterer Heterozyklus der genannten Art ankondensiert sein kann, wobei die genannten Reste 1 bis 5 insbesondere 1, 2 oder 3 Substituenten tragen können.

Als Substituenten für Alkyl seien beispielhaft Hydroxy, Alkoxy mit vorzugsweise 1 bis 4 Kohlenstoffatomen, insbesondere Methoxy und Äthoxy; Alkylcarbonyloxy mit 1 bis 7 C-Atomen, gegebenenfalls durch –OH, -Halogen, insbesondere F, Cl, Br, $C_1$–$C_4$-Alkyl, $C_1$–$C_4$-Alkoxy, Nitro und/oder Amino substituiertes Benzoyloxy, Heterocyclyl, Carbonyloxy, das sich von 5- oder 6-gliedrigen Heterocyclen ableiten, die 1 bis 3 Heteroatome (N, O, S) enthalten und im heterocyclischen Ring durch $C_1$–$C_4$-Alkyl, Chlor, Brom, Amino substituiert sein können, Amino, Monoalkylamino und Dialkylamino mit vorzugsweise 1 bis 4 Kohlenstoffatomen je Alkylrest, insbesondere Monomethylamino, Monoäthylamino, Dimethylamino und Diäthylamino, Monoacylamino, wobei der Acylrest von aliphatischen Carbonsäuren mit 1 bis 7 C-Atomen, aromatischen Carbonsäuren, insbesondere Phenylcarbonsäuren, die im Phenylrest durch –OH, -Halogen, insbesondere F, Cl, Br, $C_1$–$C_4$-Alkyl, $C_1$–$C_4$-Alkoxy, Nitro und/oder Amino substituiert sein können, heterocyclischen Carbonsäuren, die sich von 5- oder 6-gliedrigen Heterocyclen ableiten, die 1 bis 3 Heteroatome (N, O, S) enthalten und im heterocyclischen Ring durch $C_1$–$C_4$-Alkyl, Chlor, Brom, Amino substituiert sein können, abgeleitet ist; Mercapto, Alkylthio mit vorzugsweise 1 bis 4 Kohlenstoffatomen, insbesondere Methylthio und Äthylthio; Halogen, vorzugsweise Fluor, Chlor und Brom; Alkylcarbonyl mit vorzugsweise 1 bis 4 Kohlenstoffatomen im Alkylrest; Carboxy, Nitro, Cyan, Formyl, Sulfo; sowie heterocyclische Reste der oben genannten Art, und Reste von Zuckern, insbesondere von Hexosen oder Pentosen abgeleitete heterocyclische Reste, die direkt über ein Ringatom oder über eine –O–, –S– oder –NH-Brücke mit dem Alkylrest verbunden sein können, aufgeführt.

Beispiele für die vorstehend genannten Heterocyclen sind Phthalimido, Pyridyl, Thienyl, Furyl, Isoxazolyl, Thiazolyl, Glucopyranosyl, Ribofuranosyl, Oxiranyl.

Der Alkylrest kann auch einen mono-, bi- oder tricyclischen Substituenten mit vorzugsweise 3 bis 10 Kohlenstoffatomen tragen, der seinerseits durch Hydroxy, Amino, Halogen, insbesondere Fluor, Chlor, Brom, oder –COOH substituiert sein kann.

Der Alkylrest trägt bevorzugt Substituenten wie Hydroxy, Alkoxy mit 1 bis 4 Kohlenstoffatomen, Mercapto, Alkylthio mit 1 bis 4 Kohlenstoffatomen, Halogen, Nitro, Amino, Monoalkylamino mit 1 bis 4 C-Atomen und Acylamino, wobei der Acylrest von aliphatischen Carbonsäuren mit 1 bis 6 C-Atomen abgeleitet ist.

Für die cyclischen mono-, bi- oder tricyclischen Reste $R_1$, R' und R'' kommen die für die Alkylreste genannten Substituenten in Betracht.

Die Arylreste $R_5$, $R_6$ und $R_7$ können einen oder mehrere, vorzugsweise 1 bis 3, gleiche oder verschiedene Substituenten tragen.

Als Substituenten seien beispielhaft aufgeführt: Alkyl mit 1 bis 10 C-Atomen, das seinerseits wieder beispielsweise durch Chlor, Nitro oder Cyan substituiert sein kann; gegebenenfalls substituierte Alkenylreste mit 2 bis 10 Kohlenstoffatomen; Hydroxy, Alkoxy mit vorzugsweise 1 bis 4 Kohlenstoffatomen; Amino, Monoalkyl- und Dialkylamino mit vorzugsweise 1 bis 4 Kohlenstoffatomen je Alkylrest; Mercapto, Alkylthio mit vorzugsweise 1 bis 4 Kohlenstoffatomen; Carboxy, Carbalkoxy mit vorzugsweise 1 bis 4 Kohlenstoffatomen, die Sulfonsäuregruppe, Alkylsulfonyl mit vorzugsweise 1 bis 4 Kohlenstoffatomen, Arylsulfonyl, vorzugsweise Phenylsulfonyl; Aminosulfonyl-, Alkylamino- und Dialkylaminosulfonyl mit 1 bis 4 Kohlenstoffatomen je Alkylgruppe, vorzugsweise Methyl- und Dimethylaminosulfonyl; Nitro, Cyan, Formyl, Alkylcarbonylamino mit vorzugsweise 1 bis 4 Kohlenstoffatomen; Alkylcarbonyl mit 1 bis 4 Kohlenstoffatomen, Benzoyl, Benzylcarbonyl und Phenyläthylcarbonyl, wobei die zuletzt genannten Alkyl-, Phenyl-, Benzyl- und Phenyläthylreste ihrerseits wieder beispielsweise durch Chlor, Nitro oder Hydroxy substituiert sein können. Dabei ist Aryl vorzugsweise Phenyl und Naphthyl.

Die heterocyclischen Reste $R_5$, $R_6$ und $R_7$ sind bevorzugt von heteroparaffinischen, heteroaromatischen oder heteroolefinischen 5- oder 6-gliedrigen Ringen mit vorzugsweise 1 bis 3 gleichen oder verschiedenen Heteroatomen aus der Reihe Sauerstoff, Schwefel oder Stickstoff abgeleitet. Diese Ringsysteme können weitere Substituenten wie Hydroxy-, Amino- oder $C_1$–$C_4$-Alkylgruppen tragen oder an sie können Benzolkerne oder weitere vorzugsweise 6-gliedrige heterocyclische Ringe der genannten Art annelliert sein.

Besonders bevorzugte heterocyclische Reste leiten sich von Furan, Pyran, Pyrrolidin, Piperidin, Pyrazol, Imidazol, Pyrimidin, Pyridazin, Pyrazin, Triazin, Pyrrol, Pyridin, Benzimidazol, Chinolin, Isochinolin und Purin ab.

Für bevorzugte Verbindungen innerhalb der Formel (I) steht $R_3$ für Wasserstoff, Hydroxy, Sulfo, Cyano, Aminomethyl, $C_1$–$C_6$-Alkylaminomethyl und $C_1$–$C_6$-Alkylcarbonylaminomethyl.

Ganz besonders bevorzugte Verbindungen innerhalb der Formel (I) entsprechen den Formeln,

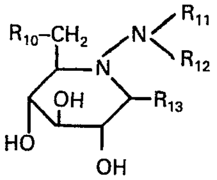

und

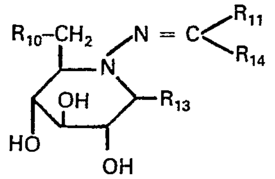

(II) (III)

worin

$R_{10}$ Wasserstoff, Hydroxy oder Methoxy,

$R_{11}$ Wasserstoff, $C_1$–$C_4$-Alkyl, $C_1$–$C_4$-Alkoxy, $C_1$–$C_4$-Alkylsulfonyl, $C_1$–$C_4$-Alkylcarbonyl oder -phenyl, insbesondere Wasserstoff, Methyl, Äthyl, Acetyl, Methylsulfonyl, Propionyl oder Phenyl,

$R_{13}$ Wasserstoff, $C_1$–$C_6$-Alkylcarbonylaminomethyl, Phenylcarbonylaminomethyl, Phenylsulfonylaminomethyl, $C_1$–$C_4$-Alkoxycarbonyl und $C_1$–$C_6$-Alkylaminocarbonyl,

$R_{12}$ Wasserstoff, gegebenenfalls durch Hydroxy, $C_1$–$C_4$-Alkoxy, Carboxy, $C_5$–$C_7$-Cycloalkyl oder Phenylsulfonylamino substituiertes $C_1$–$C_{12}$-Alkyl, $C_2$–$C_4$-Alkenyl, gegebenenfalls im Phenylring durch Halogen, insbesondere Fluor oder Chlor, $C_1$–$C_4$-Alkyl, Hydroxy, Di-$C_1$–$C_4$-alkyl oder Benzoylmethyl, $C_5$–$C_7$-Cycloalkyl, Furylmethyl, Pyridylmethyl oder Diphenylmethyl und

$R_{14}$ Wasserstoff, gegebenenfalls durch Hydroxy, $C_1$–$C_4$-Alkoxy oder Phenylsulfonylamino substituiertes $C_1$–$C_{12}$-Alkyl, $C_5$–$C_7$-Cycloalkyl, $C_2$–$C_{12}$-Alkenyl, Carboxy, gegebenenfalls durch Halogen, insbesondere Fluor und Chlor, $C_1$–$C_4$-Alkyl, Hydroxy, Nitro, Di-$C_1$–$C_4$-alkylamino, $C_1$–$C_4$-Alkoxy oder -carboxy, substituiertes Phenyl, Phenyl-$C_1$–$C_4$-Alkyl oder -benzoyl, Furyl, Pyridyl oder zusammen mit $R_{11}$ und dem doppelt gebundenen C-Atomen einen Cyclopentan- oder Cyclohexan-Ring bedeuten.

Die erfindungsgemässen Verbindungen werden erhalten, indem man Verbindungen der Formel (IV),

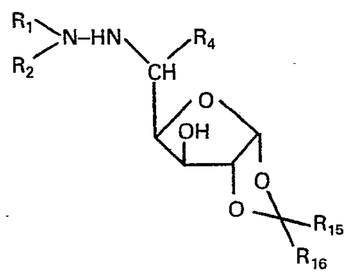

(IV)

worin

$R_{15}$ und $R_{16}$ unabhängig voneinander $C_1$–$C_4$-Alkyl oder zusammen $C_4$–$C_5$-Alkylen bedeuten und

$R_1$, $R_2$ und $R_4$ die bereits angegebene Bedeutung haben, sauer hydrolysiert und die erhaltene Verbindung der Formel (V),

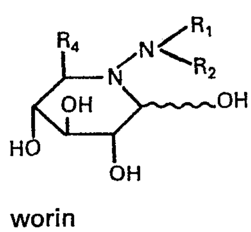

(V)

worin

$R_1$, $R_2$ und $R_4$ die bereits bekannte Bedeutung besitzen, in an sich bekannter Weise zu den Verbindungen der Formel (I), worin $R_3$ mit Ausnahme von Hydroxy die bereits genannten Bedeutungen aufweisen kann, umsetzt.

$R_{15}$ und $R_{16}$ bedeuten insbesondere Methyl oder zusammen Pentamethylen.

Verbindungen der Formel (I), worin $R_3$ mit Ausnahme von OH, SH und $NH_2$ die bekannten Bedeu-

tungen aufweist und $R_1$, $R_2$ und $R_4$ die bereits genannte Bedeutung besitzen, werden ferner dadurch erhalten, dass man Verbindungen der Formel (VI),

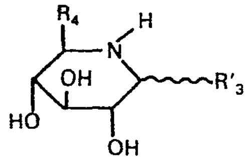

(VI)

worin

$R_4$ die bereits genannte Bedeutung hat und

$R_3'$ die Bedeutung von $R_3$ mit Ausnahme von Hydroxy, Mercapto und Amino hat, nitrosiert und reduziert und die dabei erhaltenen Verbindungen der Formel (VII),

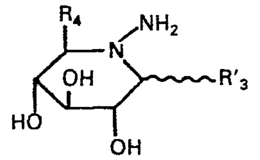

(VII)

worin

$R_3'$ und $R_4$ die genannte Bedeutung aufweisen, in üblicher Weise zu den entsprechenden Verbindungen der Formel (I), worin $R_1$ und $R_2$ die genannte Bedeutung haben, umsetzt.

Die Verbindungen der Formel (VII) können aus den Verbindungen der Formel (VI) auch dadurch erhalten werden, dass man entweder die Verbindungen der Formel (VI) mit Hydroxylamin-O-Schwefelsäure oder mit Ammoniak und Chlor in an sich bekannter Weise umsetzt.

Verbindungen der Formel (I), worin $R_3$ $OR_5$, SH, $SR_5$, $NH_2$, $NHR_5$, $NR_5R_6$, CN oder $SO_3H$ bedeutet, werden erhalten, indem man Verbindungen der Formel (V) mit entsprechenden Nukleophilen umsetzt. Die Cyanogruppe in Verbindungen der Formel (I) mit $R_3$ ist = CN kann in üblicher Weise zur Carboxylgruppe, zur Carbonsäureestergruppe, zur Carbonamidgruppe verseift oder zur Aminomäthylgruppe reduziert werden, die ihrerseits wieder alkyliert oder acyliert werden kann. Aus den Verbindungen der Formel (I), in denen $R_3$ Cyano oder Sulfo bedeutet, können die Verbindungen mit $R_3$ = OH durch Behandlung mit Basen, vorzugsweise mit Erdalkalihydroxiden, zurückgewonnen werden. Aus den Verbindungen der Formel (V) können die Verbindungen der Formel (I) mit $R_3$ = Wasserstoff durch Reduktionsmittel, beispielsweise mit Natriumcyanoborhydrid, gewonnen werden.

Verbindungen der Formel (VIII)

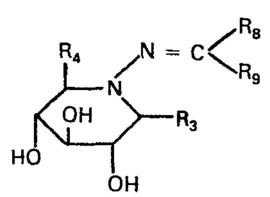

(VIII)

werden erhalten, indem man Verbindungen der Formel (VII) mit entsprechenden Aldehyden oder Ketonen in Wasser oder Alkohol, evtl. in Gegenwart einer Säule, insbesondere Essigsäure, umsetzt.

Verbindungen der Formel (VIII), in denen $R_3$ nicht OH oder SH ist, lassen sich mit Wasserstoffdonatoren, insbesondere mit $NaBH_3CN$ in Alkohol in Gegenwart von Eisessig oder mit $NaBH_4$ in Alkohol in Gegenwart von Eisessig zu Verbindungen der Formel (IX),

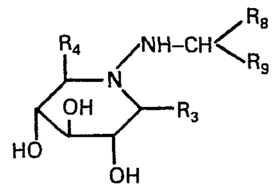

(IX)

worin

$R_3$ nicht OH oder SH ist und $R_4$, $R_8$ und $R_9$ die angegebene Bedeutung haben, reduziert.

Die Verbindungen der Formel (IX), bei denen $R_3$ nicht OH oder SH ist, können mit Aldehyden und Ketonen sowie $NaBH_3CN$ in Alkohol in Gegenwart von Säure, beispielsweise Eisessig, zu Verbindungen der Formel (I), worin $R_3$ nicht OH oder SH ist, $R_4$ die angegebene Bedeutung hat und $R_1$ und $R_2$ unabhängig voneinander für

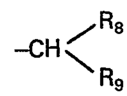

stehen, worin

$R_8$ und $R_9$ die angegebene Bedeutung haben, umgesetzt werden.

Weiterhin können die Verbindungen der Formel (IX) und die Verbindungen der Formel (VII) mit Carbonsäurechloriden und -anhydriden sowie mit Sulfonsäurechloriden besonders in Wasser oder Alkohol/Wasser-Gemischen in Gegenwart einer Base wie NaOH, $K_2CO_3$, $NaHCO_3$, insbesondere aber in Gegenwart eines basischen Ionenaustauschers, zu Verbindungen der Formel (X),

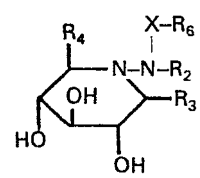

(X)

worin

X CO oder $SO_2$ bedeutet und

$R_2$, $R_3$, $R_4$ und $R_6$ die angegebene Bedeutung haben, umgesetzt werden.

Verbindungen der Formel (X) lassen sich beispielsweise mit $NaBH_4$ in Trifluoressigsäure zu Verbindungen der Formel (I), worin $R_1$ für $-CH_2-R_6$ steht und $R_2$, $R_3$, $R_4$ und $R_6$ die angegebene Bedeutung haben, reduzieren.

Aus den Verbindungen der Formeln (VII) und (IX) lassen sich durch Umsetzung mit Epoxiden und α,β-ungesättigten Carbonylverbindungen wie Acrylnitril, Verbindungen der Formel (I) erhal-

ten, worin $R_1$ eine gegebenenfalls substituierte 2-Hydroxyäthyl- oder eine gegebenenfalls substituierte 2-Cyanoäthyl-Gruppe, insbesondere die 2-Hydroxyäthyl- und die 2-Cyanoäthylgruppe bedeuten und $R_2$, $R_3$, $R_4$, $R_8$ und $R_8$ und $R_9$ die angegebenen Bedeutungen haben.

Verbindungen der Formel (XI),

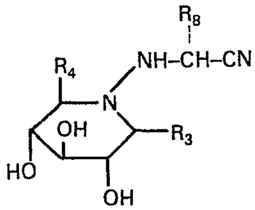

(XI)

worin

$R_3$, $R_4$ und $R_8$ die angegebene Bedeutung haben, werden erhalten, indem man Verbindungen der Formel (VII) mit 2-Hydroxynitrilen der Formel (XII),

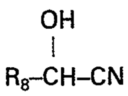

(XII)

worin

$R_8$ die angegebene Bedeutung hat,

oder indem man Verbindungen der Formel (VIII), worin $R_8$ die angegebene Bedeutung hat und $R_9$ Wasserstoff bedeutet, mit Blausäure umsetzt.

Verbindungen der Formeln (IX) und (VII), worin $R_3$ bzw. $R_3'$ eine Cyanogruppe bedeuten, die zur Aminomethylgruppe reduziert werden soll, werden zweckmässigerweise vor der Reduktion durch Umsetzung mit Carbonsäurechloriden oder Carbonsäureanhydriden, insbesondere durch Umsetzung mit Chlorameisensäure $-C_1-C_4$-Alkylestern geschützt. Die Schutzgruppe kann nach der Hydrierung oder gegebenenfalls nach einer weiteren Reaktion auf an sich bekannte Weise sauer oder basisch leicht abgespalten werden.

Die saure Hydrolyse von Verbindungen der Formel (IV) zu Verbindungen der Formel (V) wird zweckmässigerweise so durchgeführt, dass man die Produkte in der Form von Addukten der schwefeligen Säure abfängt ($R_3=SO_3H$) und aus diesen Bisulfit-Additionsprodukten die Verbindungen der Formel (I) ($R_3=OH$), wie bereits erwähnt, durch Behandlung mit Basen, vorzugsweise Erdalkalihydroxiden wie $Ca(OH)_2$, $Sr(OH)_2$ oder insbesondere $Ba(OH)_2$ in Freiheit setzt.

Die Verbindungen der Formel (IV) kann nach an sich bekannten Methoden, wie in dem folgenden Reaktionsschema gezeigt, erhalten werden, wobei die Startverbindungen der beiden Reaktionswege literaturbekannt sind.

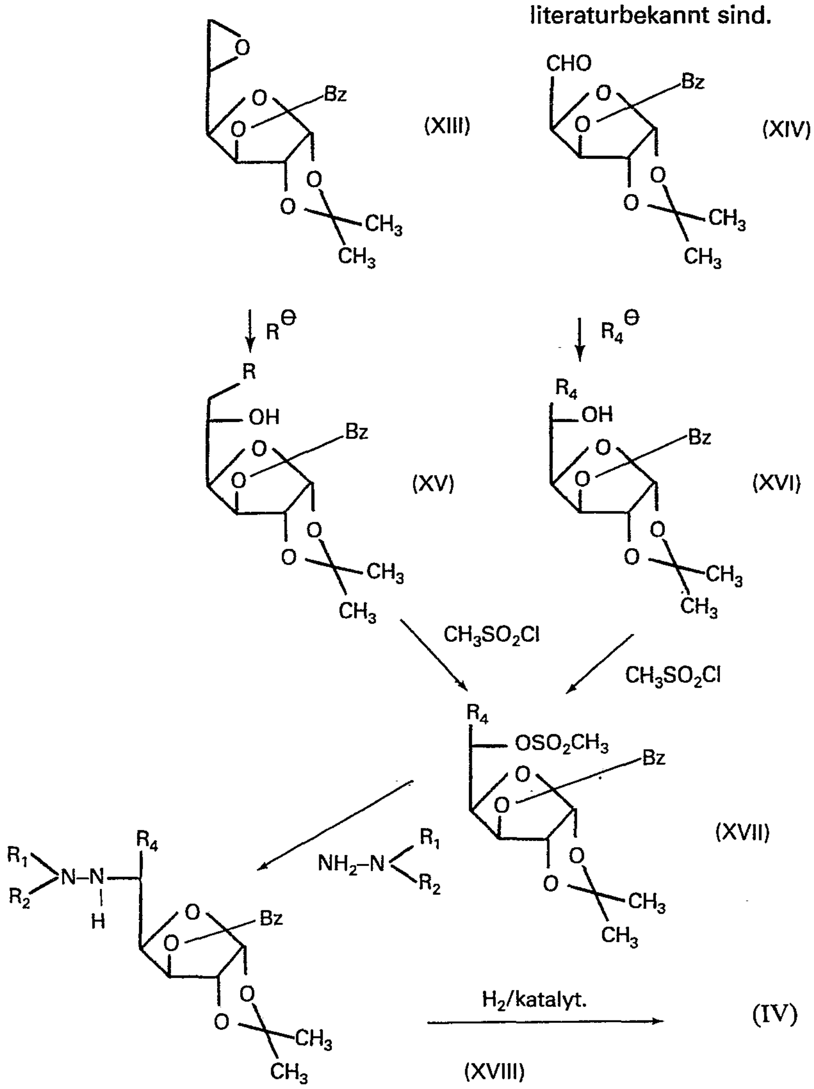


(XIII) (XIV) (XV) (XVI) (XVII) (XVIII) (IV)

In dem Reaktionsschema hat die Gruppe $-CH_2-R$ die Bedeutung $R_4$. $R_1$, $R_2$ und $R_4$ besitzen die angegebene Bedeutung, Bz steht für eine Benzylgruppe, insbesondere eine 4-Nitrobenzyl-Gruppe.

$R_4^{\ominus}$ wird z.B. als $R_4MgHal$ oder $R_4Li$, $R^{\ominus}$ als Alkoholat eingesetzt.

Die Zwischenprodukte (XV) und (XVI) sind Ido-Verbindungen. Die ebenfalls gebildeten Gluco-Isomeren von (XVI) müssen gegebenenfalls abgetrennt werden. Man kann jedoch auch so vorgehen, dass man Ido- und Gluco-Verbindungen oder Gemische der beiden in bekannter Weise, beispielsweise mit Chromtrioxid, zu den Ketonen der Formel (XIX)

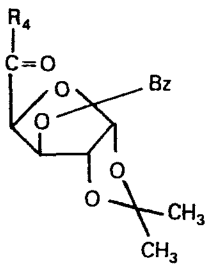

(XIX)

oxidiert, diese Ketone oder den Aldehyd (XIV) ($R_4$=H) mit den Hydrazinen der Formel

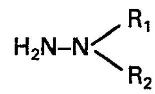

umsetzt, die dabei entstandenen Hydrazone, beispielsweise mit $NaBH_4$ oder mit NaCNBH in Alkohol/Eisessig reduziert und die Gluco-Isomeren von Ido-Isomeren chromatographisch trennt. Die Aldehydhydrazone können wie bereits vorher beschrieben mit metallorganischen Verbindungen $R_4$ MgBr oder $R_4Li$ zu einem Ido/Gluco-Gemisch der Formel (XVIII) umgesetzt werden, aus dem gegebenenfalls durch Chromatographie das Gluco-Isomere abgetrennt wird.

Die Darstellung von N-Phenylamino-1-desoxynojirimicin sei im folgenden beispielhaft beschrieben. Die dabei benutzte Ausgangsverbindung (XX) ist literaturbekannt.

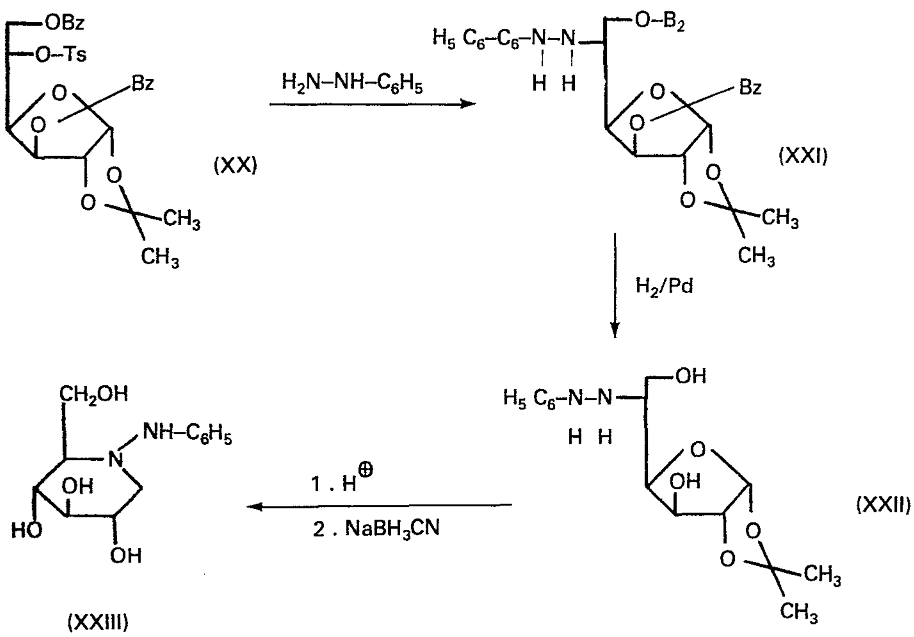


(XX) (XXI) (XXII) (XXIII)

Die Abspaltung der Isopropylidenschutzgruppe aus den Verbindungen der Formel (IV) und (XXII) erfolgt in mässig stark saurer bis schwach saurer Lösung, bevorzugt in einem pH-Bereich zwischen 1 und 4, in wässriger Lösung oder in einem mit Wasser mischbaren, wasserhaltigen organischen Lösungsmittel. Als Säure können verdünnte Mineralsäuren wie beispielsweise Schwefelsäure oder auch organische Säuren wie Essigsäure verwendet werden. Die Reaktion wird bevorzugt bei Atmosphärendruck und einer Temperatur zwischen Raumtemperatur und der Siedetemperatur des Lösungsmittels durchgeführt.

Zur Aufarbeitung des Reaktionsansatzes wird die Säure neutralisiert und als Salz oder mit Hilfe eines basischen Ionenaustauschers abgetrennt.

Die Isolierung der Verbindungen der Formel (I) mit $R_3$=OH erfolgt dann gegebenenfalls durch ein schonendes Entfernen des Lösungsmittels, beispielsweise durch Lyophilisation.

Eine bevorzugte Ausführungsform der Abspaltung der Isopropylidenschutzgruppe aus Verbindungen der Formel (IV) und (XXII) besteht darin, dass man die wässrige oder wasserhaltige alkoholische Lösung der Verbindungen der Formel (IV) und (XXII) mit $SO_2$ sättigt und mehrere Tage bei Temperaturen zwischen 20 und 80 °C aufbewahrt. Die Verbindungen der Formel (I) fallen dann als meist gut kristallisierende Bisulfitaddukte ($R_3$=$-SO_3H$) an, aus denen sich die Verbindungen der Formel (I) ($R_3$=OH) mit Hilfe von z.B. wässrigem $Ba(OH)_2$ freisetzen lassen.

Die Reduktion von Verbindungen der Formel (I) mit $R_3$=OH zu Verbindungen der Formel (I) mit $R_3$=H erfolgt durch Verwendung von Alkalimetallborhydriden, Alkalimetallcyanoborhydriden oder auch von Dialkylaminoboranen. Bevorzugt ist die Verwendung von Natriumcyanoborhydrid in Gegenwart von Eisessig in wässriger Lösung oder in einem mit Wasser mischbaren wasserhaltigen organischen Lösungsmittel, wie beispielsweise Dioxan, bei Raumtemperatur oder gegebenenfalls erhöhter Temperatur. Ganz besonders bevorzugt erfolgt die Reduktion jedoch katalytisch mit Pt oder Pd als Katalysator oder in Gegenwart von Raney-Nickel. Dabei arbeitet man bevorzugt in wässriger Lösung bei Raumtemperatur.

Die Carbonylverbindungen für die Umsetzungen von (I) mit $R_1$, $R_2$=H zu (VIII) und von (IX) zu Verbindungen der Formel (I), worin $R_1$ und $R_2$

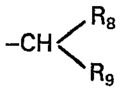

bedeuten, sind entweder bekannt oder können nach Standardverfahren hergestellt werden.

Als typische Beispiele seien im einzelnen genannt:

Gerad- oder verzweigtkettige Alkylaldehyde wie Formaldehyd, Acetaldehyd, n-Propanal, n-Butanal, 2-Methylpropanal, n-Pentanal, 2-Methylbutanal, 3-Methylbutanal, 2,2-Dimethylpropanal, n-Hexanal, 2-Äthylbutanal, n-Heptanal und n-Octanal; Alkenylaldehyde wie Propenal, 2-Methylpropenal, 2-Butenal, 2-Methyl-2-butenal, 2-Äthyl-2-hexanal; cyclische Aldehyds wie Cyclopropancarbaldehyd, Cyclopentancarbaldehyd, Cyclopentancetaldehyd, Cyclohexancarbaldehyd; Benzaldehyd, o-, m- und p-toluolcarbaldehyde und Phenylacetaldehyd; durch Hydroxy substituierte gerad- und verzweigtkettige Alkylaldehyde wie 5-Hydroxypentanal, 2-Hydroxy-3-methylbutanal, 2-Hydroxy-2-methylpropanal, 4-Hydroxybutanal, 2-Hydroxypropanal und 8-Hydroxyoctanal; durch Amino substituierte gerad- und verzweigtkettige Alkylaldehyde wie 5-Aminopentanal, 2-Aminopropanal, 3-Aminopropanal, 4-Aminobutanal, 2-Amino-3-methylbutanal, 8-Aminooctanal und mono-N-Alkylderivate davon; und durch Amino und Hydroxy disubstituierte gerad- und verzweigtkettige Alkylaldehyde wie 2-Hydroxy-5-aminopentanal, 3-Hydroxy-3-methyl-4-aminobutanal, 2-Hydroxy-4-aminobutanal, 2-Hydroxy-3-aminopropanal, 2-Hydroxy-2-methyl-3-aminopropanal, 2-Amino-3-hydroxyoctanal und mono-N-Alkylderivate davon.

Des weiteren:

Methoxy-acetaldehyd, Äthoxy-acetaldehyd, n-Propoxyacetaldehyd, i-Propoxy-acetaldehyd, n-Butoxyacetaldehyd, i-Butoxy-acetaldehyd, tert.-Butoxy-acetaldehyd, Cyclopropylmethyloxy-acetaldehyd, Cyclopropoxyacetaldehyd, 2-Methoxy-äthoxy-acetaldehyd, 2-Äthoxy-äthoxyacetaldehyd, 2-Methoxy(1-methyl-äthoxy)-acetaldehyd, 2-Äthoxy(1-methyl-äthoxy)-acetaldehyd, Phenyloxyacetaldehyd, 2-Methoxy-2-methyl-acetaldehyd, 2-Äthoxy-2-methyl-acetaldehyd, 2-n-Propoxy-2-methyl-acetaldehyd, 2-(i-Propoxy)-2-methyl-acetaldehyd, 2-(n-Butoxy)-2-methyl-acetaldehyd, 2-(i-Butoxy)-2-methyl-acetaldehyd, 2-(tert.-Butoxy)-2-methyl-acetaldehyd, 2-Cyclopropylmethyloxy-2-methyl-acetaldehyd, 2-Cyclopropyloxy-2-methyl-acetaldehyd, 2-Methoxy-äthoxy-α-methyl-acetaldehyd, 2-Äthoxy-äthoxy-α-methylacetaldehyd, 2-Methoxy-(1-methyläthoxy)-α-methyl-acetaldehyd, 2-Methoxy-2,2-dimethylacetaldehyd, 2-Äthoxy-2,2-dimethyl-acetaldehyd, 2-Cyclopropylmethyloxy-acetaldehyd, 2-ω-Butoxy-2,2-dimethyl-acetaldehyd, Methylthio-acetaldehyd, Äthylthio-acetaldehyd, n-Propylthio-acetaldehyd, i-Propylthio-acetaldehyd, Cyclopropyl-methylthio-acetaldehyd, 3-Methoxy-propanal, 3-Äthoxy-propanal, 3-n- und 3-i-propoxy-propanal, 3-n-, 3-i- und 3-tert.-Butoxy-propanal, 3-Cyclopropyloxy-propanal, 3-Cyclopropylmethyloxy-propanal, 3-Methoxy-3-methyl-propanal, 3-Äthoxy-3-methyl-propanal, 3-n- und 3-i-propoxy-3-methyl-propanal, 3-n-, 3-i- und 3-tert.-Butoxy-3-methylpropanal, 2,3- und 4-Methoxy-butanal, 2,3- und 4-Äthoxybutanal, 2-Methylthio-propanal, 2-Äthylthiopropanal, 3-Methylthio-propanal, 3-Äthylthio-propanal, 2-Methylthio-butanal, 3-Methylthio-butanal, 4-Methylthio-butanal, Furfurol, Tetrahydrofurfurol, Thiophen-2-aldehyd, 5-Bromthiophen-2-aldehyd, 5-Methylfurfurol, Pyram-carbaldehyd.

Ausserdem seien als Ketone beispielsweise genannt:

Aceton, Methyläthylketon, Methyl-n-propylketon, Diäthylketon, Methylbutylketon, Cyclopentanon, Di-n-propyl-keton, Cyclohexanon, 3-Methylcyclohexanon, 4-Methylcyclohexanon, Acetophenon, Propiophenon, Butyrophenon, Phenylaceton, p-Methoxyacetophenon, m-Nitroacetophenon.

Als Wasserstoff-Donor-Reduktionsmittel für die reduktiven Alkylierungen kann man beispielsweise Ameisensäure verwenden (Leuckart-Wallach-Reaktion). Die Ameisensäure wird in grossem Überschuss verwendet. Mit Formaldehyd als Carbonylkomponente kann die Reaktion in wässriger Lösung durchgeführt werden, mit Ketonen und weniger reaktionsfähigen Aldehyden in wasserfreier Ameisensäure. Die Reaktionstemperaturen liegen zwischen 100 und 200 °C, gegebenenfalls muss die Reaktion in einem Autoklaven durchgeführt werden.

Als Wasserstoff-Donor-Reduktionsmittel sowohl für die reduktive Alkylierung als auch für die Reduktion der Hydrazone, z.B. (VIII), werden Alkalimetallcyanoborhydride, Dialkylaminoborane und Alkalimetallborhydride verwendet. Besonders bevorzugt in dieser Verfahrensvariante ist die Verwendung von Natriumcyanoborhydrid.

Die Reaktion wird im allgemeinen bei Raumtemperatur durchgeführt. Es kann aber auch günstig sein, auf Rückflusstemperatur zu erhitzen.

Das Verfahren wird üblicherweise in einem inerten Lösungsmittel durchgeführt. Obwohl wasserfreie aprotische Lösungsmittel eingesetzt werden können (z.B. Tetrahydrofuran, wenn das Reduktionsmittel ein Dialkylaminobarane wie Dimethylaminobaran ist), wird gewöhnlich doch ein protisches Lösungsmittel verwendet. Als solches eignet sich besonders ein niederes Alkanol. Es kann aber auch Wasser oder ein wässriges niedriges Alkanol (z.B. wässriges Methanol oder Äthanol) oder andere wässrige Lösungsmittelsysteme, wie z.B. wässriges Dimethylformamid, wässriges Hexamethylphosphorsäuretriamid, wässriges Tetrahydrofuran oder wässriger Äthylenglycoldimethyläther verwendet werden.

Das Verfahren wird gewöhnlich in einem pH-Bereich von 1 bis 11 durchgeführt, bevorzugt ist ein pH-Bereich zwischen 4 und 7.

Als Beispiele für erfindungsgemässe Verbindungen seien die folgenden Wirkstoffe aufgeführt:

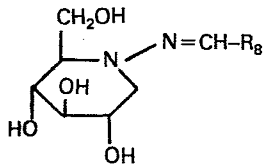

$R_8$ steht für Wasserstoff, Methyl, Äthyl, Isopropyl, n-Hexyl, n-Undecyl, Cyclopentyl, Hydroxymethyl, Methoxymethyl, 2-Phenylsulfonylaminoäthyl, Vinyl, Carboxy, Phenyl, Benzyl, 4-Chlorphenyl, 3-Fluorphenyl, 2-Methylphenyl, 4-Hydroxyphenyl, 4-Dimethylaminophenyl, 2-Nitrophenyl, Benzoyl, 3-Methoxyphenyl, 2-Carboxyphenyl, Fur-2-yl, Pyrid-3-yl, und ein Rest der Formel

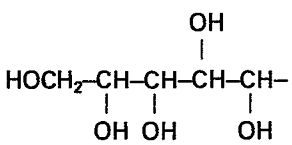

Erfindungsgemässe Verbindungen sind weiterhin solche der Formel

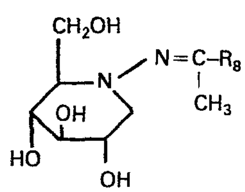

worin

$R_8$ Methyl, Phenyl oder 2-Phenyläthyl bedeutet.

Zu den erfindungsgemässen Verbindungen gehören ferner die Verbindungen der Formeln

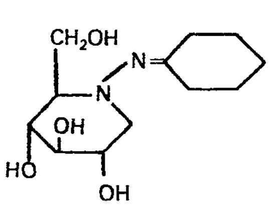

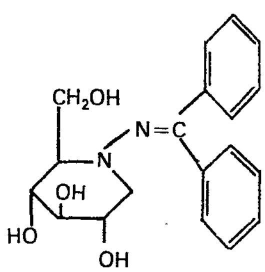

Weitere erfindungsgemässe Verbindungen entsprechen der Formel

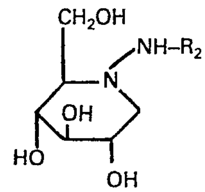

worin

$R_2$ Wasserstoff, Methyl, Äthyl, n-Propyl, iso-Butyl, n-Heptyl, n-Dodecyl, Cyclopentylmethyl, 2-Hydroxyäthyl, 2-Methoxyäthyl, 3-Phenylsulfonylaminopropyl, Allyl, Carboxymethyl, Benzyl, 2-Phenyläthyl, 4-Chlorbenzyl, 3-Fluorbenzyl, 2-Methylbenzyl, 4-Hydroxybenzyl, 4-Dimethylaminobenzyl, Phenyl, Phenylcarbonylmethyl, 3-Methoxybenzyl, 2-Carboxybenzyl, Fur-2-ylmethyl, Pyrid-3-ylmethyl, Isopropyl, Cyclohexyl, 1-Phenyläthyl, Diphenylmethyl, 1-Methyl-3-phenylpropyl oder ein Rest der Formel

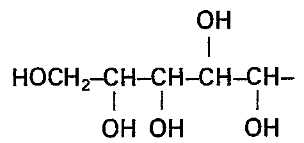

bedeuten.

Weitere erfindungsgemässe Verbindungen entsprechen der Formel

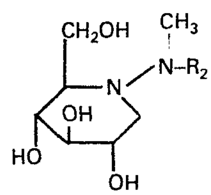

worin

$R_2$ Wasserstoff, Methyl, Äthyl, n-Propyl, iso-Butyl, n-Heptyl, n-Dodecyl, Cyclopentylmethyl, 2-Hydroxyäthyl, 2-Methoxyäthyl, 3-Phenylsulfonylaminopropyl, Allyl, Carboxymethyl, Benzyl, 2-Phenyläthyl, 4-Chlorbenzyl, 3-Fluorbenzyl, 2-Methylbenzyl, 4-Hydroxybenzyl, 4-Dimethylaminobenzyl, Phenyl, Phenylcarbonylmethyl, 3-Methoxybenzyl, 3-Carboxybenzyl, Fur-2-ylmethyl, Pyrid-3-ylmethyl, Isopropyl, Cyclohexyl, 1-Phenyläthyl, Diphenylmethyl, 1-Methyl-3-phenylpropyl oder ein Rest der Formel

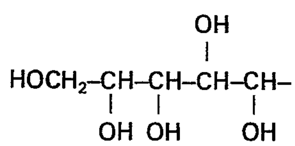

Weitere erfindungsgemässe Verbindungen entsprechen der Formel

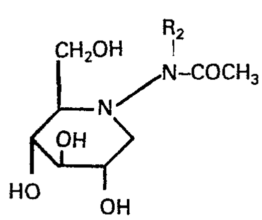

worin
$R_2$ Wasserstoff, Methyl, Äthyl, Isobutyl, Heptyl, 2-Hydroxyäthyl, Allyl, Carboxymethyl, Benzyl, 2-Phenäthyl, 4-Chlorbenzyl, 2-Methylbenzyl, 4-Hydroxybenzyl, Phenyl, Fur-2-ylmethyl, Pyrid-3-ylmethyl, Isopropyl, Cyclohexyl und 1-Phenyläthyl bedeuten.

Weitere erfindungsgemässe Verbindungen entsprechen der Formel

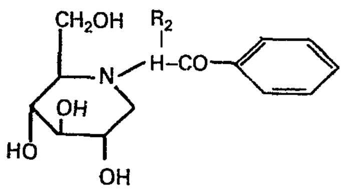

worin
$R_2$ Wasserstoff, Methyl, Äthyl, Isobutyl, Heptyl, 2-Hydroxyäthyl, Allyl, Carboxymethyl, Benzyl, 2-Phenäthyl, 4-Chlorbenzyl, 2-Methylbenzyl, 4-Hydroxybenzyl, Phenyl, Fur-2-ylmethyl, Pyrid-3-ylmethyl, Isopropyl, Cyclohexyl und 1-Phenyläthyl bedeuten.

Weitere erfindungsgemässe Verbindungen entsprechen der Formel

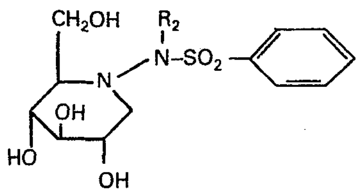

worin
$R_2$ Wasserstoff, Methyl, Äthyl, Isobutyl, Heptyl, 2-Hydroxyäthyl, Allyl, Carboxymethyl, Benzyl, 2-Phenäthyl, 4-Chlorbenzyl, 2-Methylbenzyl, 4-Hydroxybenzyl, Phenyl, Fur-2-ylmethyl, Pyrid-3-ylmethyl, Isopropyl, Cyclohexyl und 1-Phenyläthyl bedeuten.

Weitere erfindungsgemässe Verbindungen entsprechen der Formel

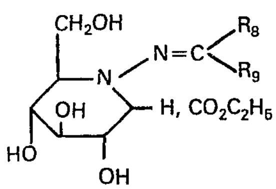

worin
$R_8$ Wasserstoff und
$R_9$ Wasserstoff, Methyl, Isopropyl, n-Hexyl, Hydroxymethyl, Vinyl, Carboxyl, Phenyl, Benzyl, 4-Chlorphenyl, 2-Methylphenyl, 4-Hydroxyphenyl, 3-Methoxyphenyl, Fur-2-yl, Pyrid-3-yl, $R_8$ Methyl und $R_9$ Methyl oder Phenyl und $R_8$ und $R_9$ zusammen mit dem C-Atom Cyclohexyl bedeuten.

Weitere erfindungesgemässe Verbindungen entsprechen der Formel

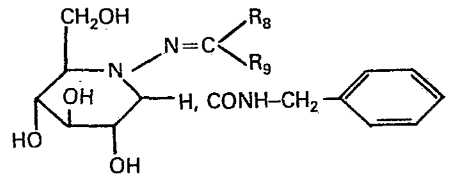

worin
$R_8$ Wasserstoff und
$R_9$ Wasserstoff, Methyl, Isopropyl, n-Hexyl, Hydroxymethyl, Vinyl, Carboxyl, Phenyl, Benzyl, 4-Chlorphenyl, 2-Methylphenyl, 4-Hydroxyphenyl, 3-Methoxyphenyl, Fur-2-yl, Pyrid-3-yl, $R_8$ Methyl und $R_9$ Methyl oder Phenyl und $R_8$ und $R_9$ zusammen mit dem C-Atom Cyclohexyl bedeuten.

Weitere erfindungsgemässe Verbindungen entsprechen der Formel

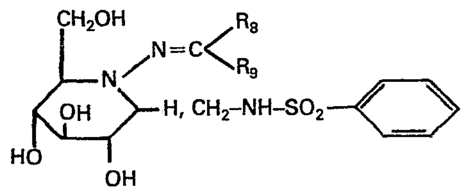

worin
$R_8$ Wasserstoff und
$R_9$ Wasserstoff, Methyl, Isopropyl, n-Hexyl, Hydroxymethyl, Vinyl, Carboxyl, Phenyl, Benzyl, 4-Chlorphenyl, 2-Methylphenyl, 4-Hydroxyphenyl, 3-Methoxyphenyl, Fur-2-yl, Pyrid-3-yl, $R_8$ Methyl und $R_9$ Methyl oder Phenyl und $R_8$ und $R_9$ zusammen mit dem C-Atom Cyclohexyl bedeuten.

Weitere erfindungsgemässe Verbindungen entsprechen der Formel

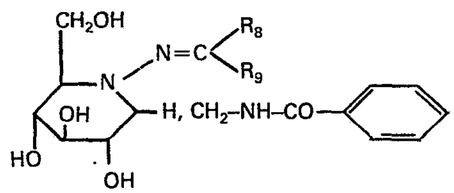

worin
$R_8$ Wasserstoff und
$R_9$ Wasserstoff, Methyl, Isopropyl, n-Hexyl, Hydroxymethyl, Vinyl, Carboxyl, Phenyl, Benzyl, 4-Chlorphenyl, 2-Methylphenyl, 4-Hydroxyphenyl, 3-Methoxyphenyl, Fur-2-yl, Pyrid-3-yl, $R_8$ Methyl und $R_9$ Methyl oder Phenyl und $R_8$ und $R_9$ zusammen mit dem C-Atom Cyclohexyl bedeuten.

Weitere erfindungsgemässe Verbindungen entsprechen der Formel

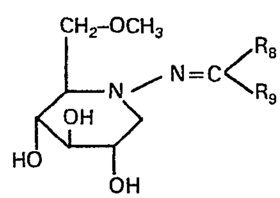

worin
$R_8$ Wasserstoff und

$R_9$ Wasserstoff, Methyl, Isopropyl, n-Hexyl, Hydroxymethyl, Vinyl, Carboxyl, Phenyl, Benzyl, 4-Chlorphenyl, 2-Methylphenyl, 4-Hydroxyphenyl, 3-Methoxyphenyl, Fur-2-yl, Pyrid-3-yl, $R_8$ Methyl und $R_9$ Methyl oder Phenyl und $R_8$ und $R_9$ zusammen mit dem C-Atom Cyclohexyl bedeuten.

Weitere erfindungsgemässe Verbindungen entsprechen der Formel

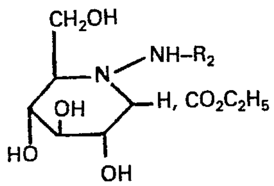

worin

$R_2$ Wasserstoff, Methyl, Äthyl, Isobutyl, n-Heptyl, 2-Hydroxyäthyl, Allyl, Carboxymethyl, Benzyl, 2-Phenäthyl, 4-Chlorbenzyl, 2-Methylbenzyl, 4-Hydroxybenzyl, Phenyl, Fur-2-ylmethyl, Pyrid-3-ylmethyl, Isopropyl, Cyclohexyl, 1-Phenyläthyl bedeuten.

Weitere erfindungsgemässe Verbindungen entsprechen der Formel

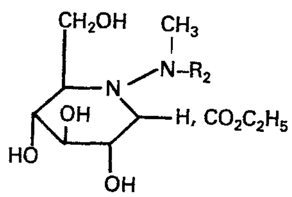

worin

$R_2$ Wasserstoff, Methyl, Äthyl, Isobutyl, n-Heptyl, 2-Hydroxyäthyl, Allyl, Carboxymethyl, Benzyl, 2-Phenäthyl, 4-Chlorbenzyl, 2-Methylbenzyl, 4-Hydroxybenzyl, Phenyl, Fur-2-ylmethyl, Pyrid-3-ylmethyl, Isopropyl, Cyclohexyl, 1-Phenyläthyl bedeuten.

Weitere erfindungsgemässe Verbindungen entsprechen der Formel

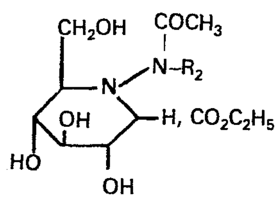

worin

$R_2$ Wasserstoff, Methyl, Äthyl, Isobutyl, n-Heptyl, 2-Hydroxyäthyl, Allyl, Carboxymethyl, Benzyl, 2-Phenäthyl, 4-Chlorbenzyl, 2-Methylbenzyl, 4-Hydroxybenzyl, Phenyl, Fur-2-ylmethyl, Pyrid-3-ylmethyl, Isopropyl, Cyclohexyl, 1-Phenyläthyl bedeuten.

Weitere erfindungsgemässe Verbindungen entsprechen der Formel

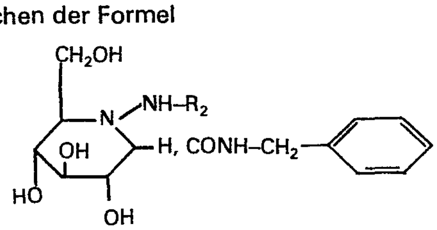

worin

$R_2$ Wasserstoff, Methyl, Äthyl, Isobutyl, n-Heptyl, 2-Hydroxyäthyl, Allyl, Carboxymethyl, Benzyl, 2-Phenäthyl, 4-Chlorbenzyl, 2-Methylbenzyl, 4-Hydroxybenzyl, Phenyl, Fur-2-ylmethyl, Pyrid-3-ylmethyl, Isopropyl, Cyclohexyl, 1-Phenyläthyl bedeuten.

Weitere erfindungsgemässe Verbindungen entsprechen der Formel

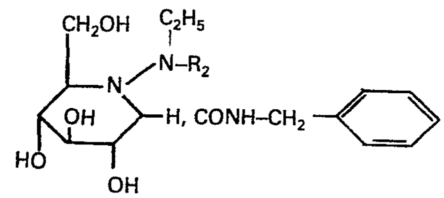

worin

$R_2$ Wasserstoff, Methyl, Äthyl, Isobutyl, n-Heptyl, 2-Hydroxyäthyl, Allyl, Carboxymethyl, Benzyl, 2-Phenäthyl, 4-Chlorbenzyl, 2-Methylbenzyl, 4-Hydroxybenzyl, Phenyl, Fur-2-ylmethyl, Pyrid-3-ylmethyl, Isopropyl, Cyclohexyl, 1-Phenyläthyl bedeuten.

Weitere erfindungsgemässe Verbindungen entsprechen der Formel

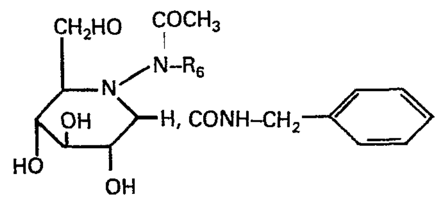

worin

$R_2$ Wasserstoff, Methyl, Äthyl, Isobutyl, n-Heptyl, 2-Hydroxyäthyl, Allyl, Carboxymethyl, Benzyl, 2-Phenäthyl, 4-Chlorbenzyl, 2-Methylbenzyl, 4-Hydroxybenzyl, Phenyl, Fur-2-ylmethyl, Pyrid-3-ylmethyl, Isopropyl, Cyclohexyl, 1-Phenyläthyl bedeuten.

Weitere erfindungsgemässe Verbindungen entsprechen der Formel

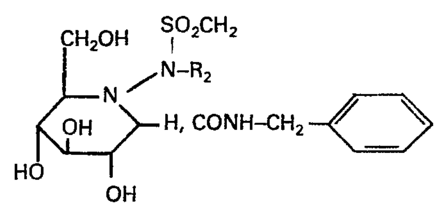

worin

$R_2$ Wasserstoff, Methyl, Äthyl, Isobutyl, n-Heptyl, 2-Hydroxyäthyl, Allyl, Carboxymethyl, Benzyl, 2-Phenäthyl, 4-Chlorbenzyl, 2-Methylbenzyl, 4-Hydroxybenzyl, Phenyl, Fur-2-ylmethyl, Pyrid-3-ylmethyl, Isopropyl, Cyclohexyl, 1-Phenyläthyl bedeuten.

Weitere erfindungsgemässe Verbindungen entsprechen der Formel

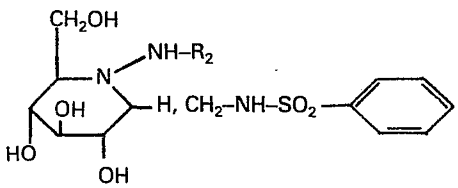

worin

$R_2$ Wasserstoff, Methyl, Äthyl, Isobutyl, n-Heptyl, 2-Hydroxyäthyl, Allyl, Carboxymethyl, Benzyl, 2-Phenäthyl, 4-Chlorbenzyl, 2-Methylbenzyl, 4-Hydroxybenzyl, Phenyl, Fur-2-ylmethyl, Pyrid-3-ylmethyl, Isopropyl, Cyclohexyl, 1-Phenyläthyl deuten.

Weitere erfindungsgemässe Verbindungen entsprechen der Formel

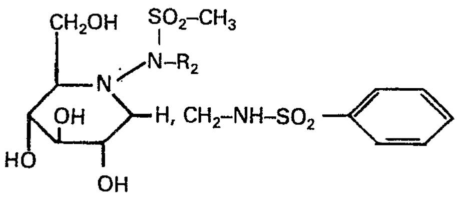

worin

$R_2$ Wasserstoff, Methyl, Äthyl, Isobutyl, n-Heptyl, 2-Hydroxyäthyl, Allyl, Carboxymethyl, Benzyl, 2-Phenäthyl, 4-Chlorbenzyl, 2-Methylbenzyl, 4-Hydroxybenzyl, Phenyl, Fur-2-ylmethyl, Pyrid-3-ylmethyl, Isopropyl, Cyclohexyl, 1-Phenyläthyl bedeuten.

Weitere erfindungsgemässe Verbindungen entsprechen der Formel

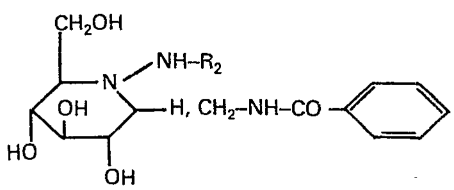

worin

$R_2$ Wasserstoff, Methyl, Äthyl, Isobutyl, n-Heptyl, 2-Hydroxyäthyl, Allyl, Carboxymethyl, Benzyl, 2-Phenäthyl, 4-Chlorbenzyl, 2-Methylbenzyl, 4-Hydroxybenzyl, Phenyl, Fur-2-ylmethyl, Pyrid-3-ylmethyl, Isopropyl, Cyclohexyl, 1-Phenyläthyl bedeuten.

Weitere erfindungsgemässe Verbindungen entsprechen der Formel

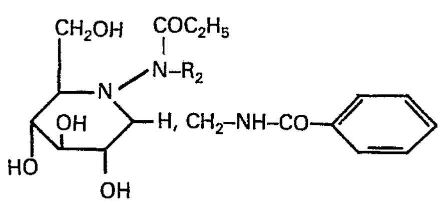

worin

$R_2$ Wasserstoff, Methyl, Äthyl, Isobutyl, n-Heptyl, 2-Hydroxyäthyl, Allyl, Carboxymethyl, Benzyl, 2-Phenäthyl, 4-Chlorbenzyl, 2-Methylbenzyl, 4-Hydroxybenzyl, Phenyl, Fur-2-ylmethyl, Pyrid-3-ylmethyl, Isopropyl, Cyclohexyl, 1-Phenyläthyl bedeuten.

Weitere erfindungsgemässe Verbindungen entsprechen der Formel

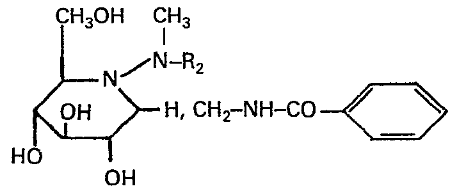

worin

$R_2$ Wasserstoff, Methyl, Äthyl, Isobutyl, n-Heptyl, 2-Hydroxyäthyl, Allyl, Carboxymethyl, Benzyl, 2-Phenäthyl, 4-Chlorbenzyl, 2-Methylbenzyl, 4-Hydroxybenzyl, Phenyl, Fur-2-ylmethyl, Pyrid-3-ylmethyl, Isopropyl, Cyclohexyl, 1-Phenyläthyl bedeuten.

Weitere erfindungsgemässe Verbindungen entsprechen der Formel

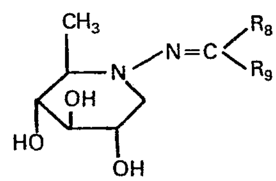

worin

$R_8$ Wasserstoff und

$R_9$ Wasserstoff, Methyl, Äthyl, Isopropyl, n-Heptyl, n-Undecyl, n-Cyclopentyl, Hydroxymethyl, Methoxymethyl, 2-Phenylsulfonylaminoäthyl, Vinyl, Carboxy, Phenyl, Benzyl, 4-Chlorphenyl, 3-Fluorphenyl, 2-Methylphenyl, 4-Hydroxyphenyl, 4-Dimethylaminophenyl, 2-Nitrophenyl, Benzoyl, 3-Methoxyphenyl, 2-Carboxyphenyl, Fur-2-yl, Pyrid-3-yl, $R_8$ Methyl und $R_9$ Methyl, Phenyl oder 2-Phenäthyl, $R_8$ und $R_9$ Phenyl, $R_8$ und $R_9$ zusammen mit dem C-Atom Cyclohexyl und $R_8$ Wasserstoff und $R_9$ einen Rest der Formel

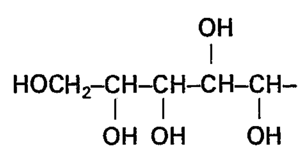

Weitere erfindungsgemässe Verbindungen entsprechen der Formel

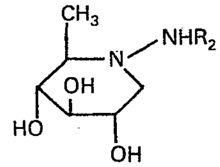

worin

$R_2$ Wasserstoff, Methyl, Äthyl, n-Propyl, Isobutyl, n-Heptyl, n-Dodecyl, Cyclopentylmethyl, 2-Hydroxyäthyl, 2-Methoxyäthyl, 3-Phenylsulfonylaminopropyl, Allyl, Carboxymethyl, Benzyl, 2-Phenyläthyl, 4-Chlorbenzyl, 3-Fluorbenzyl, 2-Methylbenzyl, 4-Hydroxybenzyl, 4-Dimethylaminobenzyl, Phenyl, Phenylcarbonylmethyl, 3-Methoxybenzyl, 2-Carboxybenzyl, Fur-2-ylmethyl, Pyrid-3-ylmethyl, Isopropyl, Cyclohexyl, 1-Phenyläthyl, Diphenylmethyl, 1-Methyl-3-Phenylpropyl oder einen Rest der Formel

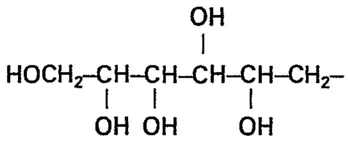

bedeuten.

Weitere erfindungsgemässe Verbindungen entsprechen der Formel

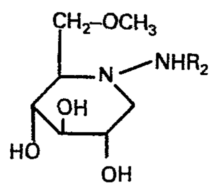

worin

$R_2$ Wasserstoff, Methyl, Äthyl, Isobuyl, n-Heptyl, 2-Hydroxyäthyl, Allyl, Carboxymethyl, Benzyl, 2-Phenäthyl, 4-Chlorbenzyl, 2-Methylbenzyl, 4-Hydroxybenzyl, Phenyl, Fur-2-ylmethyl, Pyrid-3-ylmethyl, Isopropyl, Cyclohexyl oder 1-Phenyläthyl bedeuten.

Die erfindungsgemässen Substanzen sind Inhibitoren der α-Glucosidasen des Gastrointestinaltrakts. Sie können daher die Verdauung von Kohlenhydraten hemmen. Ausserdem hemmen sie die Absorption von Cholesterin und Triglyceriden aus dem Darm. Sie sind daher geeignet als Therapeutika für:

Prädiabetes, Gastritis, Obstipation, Meterorismus, Flatulenz, gastrointestinale Infektionen, Hypertension, Karies, Atherosklerose und besonders Adipositas, Diabetes und Hyperlipoproteinämie.

Zur Verbreiterung des Wirkungsspektrums kann es sich empfehlen, Inhibitoren für Glykosidhydrolasen, die sich gegenseitig in ihrer Wirkung ergänzen, zu kombinieren, sei es, dass es sich um Kombinationen der erfindungsgemässen Inhibitoren untereinander oder um Kombinationen der erfindungsgemässen Inhibitoren mit bereits bekannten handelt. So kann es beispielsweise zweckmässig sein, erfindungsgemässe Saccharase-Inhibitoren mit bereits bekannten Amylase-Inhibitoren zu kombinieren.

Vorteilhaft sind in manchen Fällen auch Kombinationen der erfindungsgemässen Inhibitoren mit bekannten oralen Antidiabetica (β-cytotrope Sulfonylharnstoffderivate und/oder blutzuckerwirksame Biguanide), mit blutlipidsenkenden Wirkstoffen wie z.B. Clofibrat, Nicotinsäure, Cholestyramin mit bakteriziden Therapeutika sowie mit Antihypertensiva.

Die Verbindungen können ohne Verdünnung, z.B. als Pulver oder in einer Gelatinehülle oder in Kombination mit einem Trägerstoff in einer pharmazeutischen Zusammensetzung appliziert werden.

Pharmazeutische Zubereitungen können eine grössere oder kleinere Menge des Inhibitors enthalten, z.B. 0,1% bis 99,5%, in Kombination mit einem pharmazeutisch verträglichen nichttoxischen, inerten Trägerstoff, wobei der Trägerstoff eine oder mehrere feste, halbfeste oder flüssige Verdünnungsmittel, Füllstoffe und/oder nichttoxisches, inertes und pharmazeutisch-verträgliches Formulierungshilfsmittel enthalten kann. Solche pharmazeutischen Zubereitungen liegen vorzugsweise in Form von Dosierungseinheiten vor, d.h. physikalisch-diskreten, eine bestimmte Menge des Inhibitors enthaltenden Einheiten, die einem Bruchteil oder einem Vielfachen der Dosis entsprechen, die zur Herbeiführung der gewünschten Hemmwirkung erforderlich sind. Die Dosierungseinheiten können 1, 2, 3, 4 oder mehr Einzeldosen oder 1/2, 1/3 oder 1/4 einer Einzeldosis enthalten. Eine Einzeldosis enthält vorzugsweise eine genügende Menge Wirkstoff, um bei einer Applikation gemäss eines vorher bestimmten Dosierungsschemas einer oder mehrerer Dosierungseinheiten die gewünschte Hemmwirkung zu erzielen, wobei eine ganze, eine halbe, oder ein Drittel oder ein Viertel der Tagesdosis gewöhnlich zu allen Haupt- und Nebenmahlzeiten am Tage verabreicht wird.

Andere therapeutische Mittel können auch eingenommen werden. Obgleich die Dosierung und das Dosierungsschema in jedem Fall sorgsam abgewogen werden sollte, unter Anwendung gründlichen fachmännischen Urteils und unter Beachtung des Alters, des Gewichts und des Zustands des Patienten, der Art und der Schwere der Erkrankung, wird die Dosierung gewöhnlich in einem Bereich zwischen etwa 1 bis etwa $1 \times 10^4$ SIE/kg des Körpergewichtes pro Tag liegen. In manchen Fällen wird man dabei eine ausreichende therapeutische Wirkung mit einer geringeren Dosis erreichen, während in anderen Fällen eine grössere Dosis erforderlich sein wird.

Orale Applikation kann unter Verwendung fester und flüssiger Dosierungseinheiten durchgeführt werden, wie z.B. Pulver, Tabletten, Dragees, Kapseln, Granulate, Suspensionen, Lösungen und dergleichen.

Pulver wird durch Zerkleinerung der Substanz in einer geeigneten Grösse und Vermischen mit einem ebenfalls zerkleinerten pharmazeutischen Trägerstoff hergestellt. Obgleich ein essbares Kohlehydrat, wie z.B. Stärke, Lactose, Saccharose oder Glucose normalerweise zu diesem Zwecke Verwendung findet und auch hier benutzt werden kann, ist es wünschenswert ein nicht metabolisierbares Kohlenhydrat, wie z.B. ein Cellulosederivat, zu benutzen.

Süssmittel, Geschmackszusätze, Konservierungsstoffe, Dispergiermittel und Färbemittel können auch mitverwendet werden.

Die Kapseln können durch Zubereitung der oben beschriebenen Pulvermischung und durch Fällung bereits gebildeter Gelatinehüllen hergestellt werden. Die Pulvermischung kann man vor dem Füllvorgang mit Gleitmitteln, wie z.B. Kieselgel, Talkum, Magnesiumstearat, Calciumstearat oder festem Polyäthylenglykol versetzen. Die Mischung kann man ebenfalls mit einem Desintegrator oder Lösungsvermittler, wie z.B. Agar-Agar, Calciumcarbonat oder Natriumcarbonat versetzen, um bei Einnahme der Kapsel die Zugänglichkeit des Inhibitors zu verbessern.

Die Anfertigung der Tabletten erfolgt, z.B. durch Herstellung einer Pulvermischung, grob oder feinkörnig, und Hinzufügung eines Gleitmittels und Desintegrators. Aus dieser Mischung formt man Tabletten. Eine Pulvermischung bereitet man vor durch Mischung der Substanz, welche in geeigneter Weise zerkleinert wurde und ergänzt ein Verdünnungsmittel oder eine andere Trägersubstanz wie oben beschrieben. Gegebenenfalls fügt man ein Bindemittel hinzu: z.B. Carboxymethylcellulose, Alginate, Gelatine oder Polyvinylpyrrolidone, einen Lösungsverzögerer, wie z.B. Paraffin, einen Resorptionsbeschleuniger, wie z.B. ein quarternäres Salz und/oder ein Adsorptionsmittel, wie z.B. Bentonit, Kaolin oder Dicalciumphosphat. Die Pulvermischung kann granuliert werden zusammen mit einem Bindemittel, wie z.B. Sirup, Stärkepaste, Akazienschleim, oder Lösungen aus Zellulose- oder Polymerenmaterialien. Danach presst man das Produkt durch ein grobes Sieb. Als Alternative hierzu kann man die Pulvermischung durch eine Tablettenmaschine laufen lassen und die sich ergebenden ungleichmässig geformten Stücke bis auf Korngrösse zerkleinern. Damit die entstandenen Körper nicht in den tablettenbildenden Düsen stecken bleiben, kann man sie mit einem Gleitmittel versetzen, wie z.B. Stearinsäure, Stearatsalz, Talkum oder Mineralöl. Diese gleitfähig gemachte Mischung wird dann in Tablettenform gepresst. Die Wirkstoffe können auch mit freiliessenden inerten Trägerstoffen vereinigt werden und direkt in Tablettenform gebracht werden unter Auslassung der Granulat- oder Zerstückelungsschritte. Man kann das Produkt mit einer klaren oder opaken Schutzhülle versehen, z.B. einem Überzug aus Schellack, einem Überzug aus Zucker oder Polymersubstanzen und einer polierten Hülle aus Wachs. Farbstoffe können diesen Überzügen beigefügt werden, damit zwischen den verschiedenen Dosierungseinheiten unterschieden werden kann.

Die oral zu verabreichenden Zubereitungsformen, wie z.B. Lösungen, Syrup und Elixire, lassen sich in Dosierungseinheiten herstellen, so dass eine bestimmte Menge Präparat eine bestimmte Menge Wirkstoff enthält. Syrup kann so hergestellt werden, dass der Wirkstoff in einer wässrigen Lösung, welche geeignete Geschmacksstoffe enthält, gelöst wird; Elixire werden unter Verwendung nichttoxischer, alkoholischer Trägerstoffe erhalten. Suspensionen kann man durch Dispergieren oder Verbindung in einem nichttoxischen Trägerstoff darstellen. Lösungsvermittler und Emulgiermittel, wie z.B. äthoxylierte Isostearylalkohole und Polyoxyäthylensorbitester, Konservierungsmittel, geschmacksverbessernde Zusätze wie z.B. Pfefferminzöl oder Saccharin und dergleichen können auch zugegeben werden.

Dosierungsvorschriften können auf der Kapsel angegeben werden. Überdies kann die Dosierung so abgesichert sein, dass der Wirkstoff verzögert abgegeben wird, z.B. durch Einhalten des Wirkstoffes in Polymerensubstanzen, Wachsen oder dergleichen.

Zusätzlich zu den oben erwähnten pharmazeutischen Zusammensetzungen lassen sich auch diese Wirkstoffe enthaltende Lebensmittel herstellen, beispielsweise Zucker, Brot, Kartoffelprodukte, Fruchtsaft, Bier, Schokolade und andere Konfektartikel, und Konserven, wie z.B. Marmelade, wobei zu diesen Produkten eine therapeutisch-wirksame Menge mindestens eines der erfindungsgemässen Inhibitoren gegeben wird.

Die unter Verwendung der erfindungsgemässen Wirkstoffe hergestellten Nahrungsmittel eignen sich sowohl zur Diät bei Patienten, die an Stoffwechselstörungen leiden als auch zur Ernährung gesunder Personen im Sinne einer Stoffwechselstörungen vorbeugenden Ernährungsweise.

Die erfindungsgemässen Inhibitoren weisen weiterhin die Eigenschaft auf, in Tieren das Verhältnis des Anteiles an unerwünschtem Fett zum Anteil des erwünschten fettarmen Fleisches (mageres Fleisch) zugunsten des mageren Fleisches in hohem Masse zu beeinflussen. Dies ist von besonderer Bedeutung für die Aufzucht und Haltung von landwirtschaftlichen Nutztieren, z.B. in der Schweinemast, aber auch von erheblicher Bedeutung für die Aufzucht und Haltung von sonstigen Nutztieren und Ziertieren. Die Verwendung der Inhibitoren kann weiterhin zu einer erheblichen Rationalisierung der Fütterung der Tiere führen, sowohl zeitlich, mengenmässig wie auch qualitätsmässig. Da die Inhibitoren eine gewisse Verzögerung der Verdauung bewirken, wird die Verweildauer der Nährstoffe im Verdauungstrakt verlängert, wodurch eine mit weniger Aufwand verbundene ad libitum-Fütterung ermöglicht wird. Weiterhin ergibt sich bei der Verwendung der erfindungsgemässen Inhibitoren in vielen Fällen eine erhebliche Einsparung von wertvollem Proteinfutter.

Die Wirkstoffe können somit praktisch in allen Bereichen der Tierernährung als Mittel zur Reduzierung des Fettansatzes sowie der Einsparung von Futtereiweiss verwendet werden.

Die Wirksamkeit der Wirkstoffe ist hierbei weitgehend unabhängig von der Art und dem Geschlecht der Tiere. Besonders wertvoll erweisen sich die Wirkstoffe bei Tierarten, die überhaupt oder in bestimmten Lebensabschnitten zu stärkerer Fetteinlagerung neigen.

Als Tiere, bei denen die Inhibitoren zur Reduzierung des Fettansatzes und/oder zur Einsparung von Futtereiweiss eingesetzt werden können, seien beispielsweise folgende Nutz- und Ziertiere genannt: Warmblüter wie Rinder, Schweine, Pferde, Schafe, Ziegen, Katzen, Hunde, Kaninchen, Pelztiere, z.B. Nerze, Chinchilla, andere Ziertiere, z.B. Meerschweinchen und Hamster, Labor- und

Zootiere, z.B. Ratten, Mäuse, Affen usw., Geflügel, z.B. Broiler, Hühner, Gänse, Enten, Truthähne, Tauben, Papageien und Kanarienvögel und Kaltblüter, wie Fische, z.B. Karpfen und Reptilien, z.B. Schlangen.

Die Menge der Wirkstoffe, die den Tieren zur Erreichung des gewünschten Effektes verabreicht wird, kann wegen der günstigen Eigenschaften der Wirkstoffe weitgehend variiert werden. Sie liegt vorzugsweise bei etwa 0,5 mg bis 2,5 g, insbesondere 10 bis 100 mg/kg Futter pro Tag. Die Dauer der Verabreichung kann von wenigen Stunden oder Tagen bis zu mehreren Jahren betragen. Die passende Menge Wirkstoff sowie die passende Dauer der Verabreichung stehen in engem Zusammenhang mit dem Fütterungsziel. Sie hängen insbesondere von der Art, dem Alter, dem Geschlecht, dem Gesundheitszustand und der Art der Haltung der Tiere ab und sind durch jeden Fachmann leicht zu ermitteln.

Die erfindungsgemässen Wirkstoffe werden den Tieren nach den üblichen Methoden verabreicht. Die Art der Verabreichung hängt insbesondere von der Art, dem Verhalten und dem Allgemeinzustand der Tiere ab. So kann die Verabreichung einmal oder mehrmals täglich, in regelmässigen und unregelmässigen Abständen, oral erfolgen. Aus Zweckmässigkeitsgründen ist in den meisten Fällen eine orale Verabreichung, insbesondere im Rythmus der Nahrungs- und/oder Getränkeaufnahme der Tiere, vorzuziehen.

Die Wirkstoffe können als reine Stoffe oder in formulierter Form verabreicht werden, wobei die formulierte Form sowohl als Premix, also in Mischung mit nichttoxischen inerten Trägerstoffen beliebiger Art, als auch als Teil einer Gesamtration in Form eines Beifutters bzw. als Mischungsbestandteil eines alleinigen Mischfutters zu verstehen ist. Mit eingeschlossen ist auch die Applikation geeigneter Zubereitungen über das Trinkwasser.

Die Wirkstoffe können gegebenenfalls in formulierter Form auch zusammen mit anderen Nähr- und Wirkstoffen, z.B. Mineralsalzen, Spurenelementen, Vitaminen, Eiweissstoffen, Energieträgern (z.B. Stärke, Zucker, Fette), Farbstoffen und/oder Geschmacksstoffen oder anderen Futterzusatzstoffen, wie z.B. Wachstumsförderern, in geeigneter Form verabreicht werden. Die Wirkstoffe können den Tieren vor, während oder nach der Nahrungsaufnahme gegeben werden.

Empfehlenswert ist die orale Verabreichung zusammen mit dem Futter und/oder Trinkwasser, wobei je nach Bedarf die Wirkstoffe der Gesamtmenge oder zu Teilen des Futters und/oder Trinkwassers zugegeben werden.

Die Wirkstoffe können nach üblichen Methoden durch einfaches Mischen als reine Stoffe, vorzugsweise in fein verteilter Form oder in formulierter Form in Mischung mit essbaren, nichttoxischen Trägerstoffen, gegebenenfalls auch in Form eines Premix oder eines Futterkonzentrates, dem Futter und/oder dem Trinkwasser beigefügt werden.

Das Futter und/oder Trinkwasser kann beispielsweise die erfindungsgemässen Wirkstoffe in einer Konzentration von etwa 0,001 bis 5,0%, insbesondere 0,02 bis 2,0% (Gewicht) enthalten. Die optimale Höhe der Konzentration des Wirkstoffs im Futter und/oder Trinkwasser ist insbesondere abhängig von der Menge der Futter- und/oder Trinkwasseraufnahme der Tiere und kann durch jeden Fachmann leicht ermittelt werden.

Die Art des Futters und seine Zusammensetzung ist hierbei ohne Belang. Es können alle gebräuchlichen, handelsüblichen oder speziellen Futterzusammensetzungen verwendet werden, die vorzugsweise das übliche, für eine ausgewogene Ernährung notwendige Gleichgewicht aus Energie-und Eiweissstoffen, einschliesslich Vitaminen und Mineralstoffen enthalten. Das Futter kann sich beispielsweise zusammensetzen aus pflanzlichen Stoffen, z.B. Ölkuchenschroten, Getreideschroten, Getreidenebenprodukten, aber auch aus Heu, Gärfutter, Rüben und anderen Futterpflanzen, aus tierischen Stoffen, z.B. Fleisch- und Fischprodukten, Knochenmehl, Fetten, Vitaminen, z.B. A, D, E, K und B-Komplex sowie speziellen Proteinquellen, z.B. Hefen sowie bestimmten Aminosäuren und Mineralstoffen und Spurenelementen, wie z.B. Phosphor und Eisen, Zink, Mangan, Kupfer, Kobalt, Jod usw.

Premixe können vorzugsweise etwa 0,1 bis 50%, insbesondere 0,5 bis 5,0% (Gewicht) z.B. N-Amino-1-desoxynojirimycin neben beliebigen essbaren Trägerstoffen und/oder Mineralsalzen, z.B. kohlensaurem Futterkalk enthalten und werden nach den üblichen Mischmethoden hergestellt.

Mischfutter enthalten vorzugsweise 0,001 bis 5,0%, insbesondere 0,02 bis 2,0% (Gewicht) beispielsweise an N-Amino-1-desoxynojirimycin neben den üblichen Rohstoffkomponenten eines Mischfutters, z.B. Getreideschrote oder -nebenprodukte, Ölkuchenschrote, tierisches Eiweiss, Mineralien, Spurenelementen und Vitamine. Sie können nach den üblichen Mischmethoden hergestellt werden.

Vorzugsweise in Premixen und Mischfuttermitteln können die Wirksotffe gegebenenfalls auch durch ihre Oberfläche bedeckenden geeigneten Mittel, z.B. mit nichttoxischen Wachsen oder Gelatine vor Luft, Licht und/oder Feuchtigkeit geschützt werden.

Beispiel für die Zusammensetzung eines fertigen Mischfutters für Geflügel, das einen erfindungsgemässen Wirkstoff enthält:

200 g Weizen, 340 g Mais, 360,3 g Sojaschrot, 60 g Rindertalg, 15 g Dicalciumphosphat, 10 g Calciumcarbonat, 4 g jodiertes Kochsalz, 7,5 g Vitamin-Mineral-Mischung und 3,2 g Wirkstoff-Premix ergeben nach sorgfältigem Mischen 1 kg Futter.

Die Vitamin-Mineral-Mischung besteht aus:

6000 I.E. Vitamin A, 1000 I.E. Vitamin $D_3$, 10 mg Vitamin E, 1 mg Vitamin $K_3$, 3 mg Riboflavin, 2 mg Pyridoxin, 20 mg Vitamin $B_{12}$, 5 mg Calciumpantothenat, 30 mg Nikotinsäure, 200 mg Cholinchlorid, 200 mg Mn $SO_L \times H_2O$, 140 mg Zn $SO_4 \times 7H_2O$, 100 mg Fe $SO_4 \times 7H_2O$ und 20 mg Cu $SO_4 \times 5H_2O$. Der Wirkstoff-Premix enthält z.B. N-Amino-1-desoxynojirimycin in der gewünschten Menge,

z.B. 1600 mg und zusätzlich 1 g DL-Methionin sowie so viel Sojabohnenmehl, dass 3,2 g Premix entstehen.

Beispiel für die Zusammensetzung eines Schweinemischfutters, das einen Wirkstoff der Formel (I) enthält:

630 g Futtergetreideschrot (zusammengesetzt aus 200 g Mais-, 150 g Gerste-, 150 g Hafer- und 130 g Weizenschrot), 80 g Fischmehl, 60 g Sojaschrot, 58,8 g Tapickamehl, 38 g Bierhefe, 50 g Vitamin-Mineral-Mischung für Schweine (Zusammensetzung z.B. wie beim Kükenfutter), 30 g Leinkuchenmehl, 30 g Maiskleberfutter, 10 g Sojaöl, 10 g Zuckerrohrmelasse und 2 g Wirkstoff-Premix (Zusammensetzung z.B. beim Kükenfutter) ergeben nach sorgfältigem Mischen 1 kg Futter.

Die angegebenen Futtergemische sind vorzugsweise zur Aufzucht und Mast von Küken bzw. Schweinen abgestimmt, sie können jedoch in gleicher oder ähnlicher Zusammensetzung auch zur Aufzucht und Mast anderer Tiere verwendet werden.

Die Inhibitoren können einzeln oder aber auch in beliebigen Mischungen untereinander verwendet werden.

Saccharase-Inhibitionstest in vitro

Der Saccharase-Inhibitionstest in vitro ermöglicht die Bestimmung der enzyminhibitorischen Aktivität der erfindungsgemässen Substanzen durch den Vergleich der Aktivität des solubilisierten intestinalen Disaccharidasen-Komplexes in Gegenwart bzw. in Abwesenheit (sog. 100%-Wert) des Inhibitors. Als Substrat, welches die Spezifität des Inhibitionstestes bestimmt, dient dabei eine praktisch Glucosefreie Saccharose (Glucose < 100 ppm); die Enzymaktivitätsbestimmung basiert auf der spektrophotometrischen Bestimmung freigesetzter Glucose mittels Glucose-Dehydrogenase und Nicotinamid-adenin-dinucleotid als Cofaktor.

Eine Saccharase-Inhibitor-Einheit (SIE) ist definiert als diejenige inhibitorische Aktivität, welche in einem definierten Testansatz eine vorgegebene saccarolytische Aktivität um eine Einheit (Saccharase-Einheit = SE) reduziert; die Saccharase-Einheit ist dabei als diejenige Enzymaktivität definiert, welche unter vorgegebenen Bedingungen ein µmol Saccharose pro Minute spaltet und damit zur Freisetzung von je ein µmol Glucose, welche im Test bestimmt wird, und Fructose, welche im Test nicht erfasst wird, führt.

Der intestinale Disaccharidasen-Komplex wird aus Schweinedünndarm-Mucosa durch tryptische Verdauung, Fällung aus 66% Äthanol bei −20 °C, Aufnehmen des Präcipitates in 100 mM Phosphat-Puffer, pH 7,0 und abschliessende Dialyse gegen denselben Puffer gewonnen.

10 µl eine Probelösung, die so angesetzt ist, dass die Extinktion des Testansatzes mindestens 10%, jedoch nicht mehr als 25% unter der des 100%-Wertes liegt, werden mit 100 µl einer Verdünnung des intestinalen Disaccharidasen-Komplexes in 0,1 M Maleinat-Puffer, pH 6,25, versetzt und für 10 Minuten bei 37 °C vorinkubiert. Die Verdünnung des Disaccharidasen-Komplexes ist auf eine Aktivität von 0,1 SE/ml einzustellen.

Anschliessend wird die saccharolytische Reaktion durch Zugabe von 100 µl einer 0,4 M Lösung von Saccharose («SERVA 35579») in 0,1 M Maleinat-Puffer, pH 6,25 gestartet und nach einer Inkubationsdauer von 20 Minuten bei 37 °C durch die Zugabe von 1 ml Glucose-Dehydrogenase-Reagenz (1 Fläschen Glucose-Dehydrogenase-Mutarotase-Gemisch lyophiliert («MERCK 14053») und 331,7 mg β-Nicotinamid-adenin-dinucleotid (freie Säure, «BOEHRINGER» Reinheitsgrad I) in 250 ml 0,5 M Tris-Puffer, pH 7,6 gelöst) abgestoppt. Zum Nachweis der Glucose wird 30 Minuten bei 37 °C inkubiert und schliesslich bei 340 nm gegen einen Reagenzienblank (mit Enzym, jedoch ohne Saccharose) photometriert.

Die Berechnung der Hemmaktivität von Inhibitoren ist dadurch erschwert, dass schon geringfügige Änderungen im Testsystem, beispielsweise ein geringfügig von Bestimmung zu Bestimmung variierender 100%-Wert, von nicht mehr zu vernachlässigendem Einfluss auf das Testergebnis sind. Man umgeht diese Schwierigkeiten, indem man bei jeder Bestimmung einen Standard mitlaufen lässt; als Standard dient ein Saccharose-Inhibitor der Formel $C_{25}H_{43}O_{18}N$, welcher eine spezifische Hemmaktivität von 77 700 SIE/g aufweist und bei eingesetzten Mengen von 10 bis 20 ng im Test zu einer Hemmung von oben spezifizierter Grössenordnung führt. Bei Kenntnis der Differenz der Extinktionen bei 340 nm von 100%-Wert und durch Standard gehemmtem Ansatz lässt sich aus der Extinktionsdifferenz von 100%-Wert und durch die Probelösung gehemmtem Ansatz unter Berücksichtigung der eingesetzten Menge an Inhibitor in bekannter Weise dessen spezifische Hemmaktivität errechnen, ausgedrückt in Saccharase-Inhibitor-Einheiten pro Gramm (SIE/g).

Beispiel 1

N-Amino-1-desoxynojirimycin

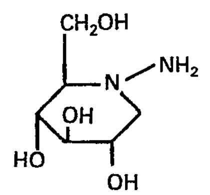

Zu 82 g 1-Desoxynojirimycin in 600 ml 2 N HCl gibt man bei 0 °C portionsweise 69 g Natriumnitrit und rührt 6 Stunden bei Raumtemperatur. Dann wird mit 1 l konzentrierter Ammoniaklösung alkalisch gestellt, unter Eiskühlung mit 166 g Zinkpulver portionsweise versetzt, über Nacht bei Raumtemperatur gerührt und 3 Stunden am Rückfluss gekocht. Nach dem Abkühlen filtriert man den Niederschlag ab, engt das Volumen im Vakuum auf 200 ml ein, filtriert den Niederschlag ab, wäscht mit Wasser und kocht das Filtrat mit 800 ml Äthanol auf. Aus der heiss filtrierten Lösung kristallisiert rohes N-Amino-1-desoxynojirimycin aus, das noch 2 ml aus Äthanol/Wasser umkristallisiert wird. Ausbeute 35 g, Schmelzpunkt 186 bis 189 °C.

Die gesammelten Mutterlaugen werden eingedampft und der Rückstand an 500 g Kieselgel (Korngrösse 0,063 bis 0,2 mm) mit Äthanolwasser (10:2) als Laufmittel chromatographiert. Man erhält weitere 27 g Ausbeute.

Beispiel 2
N-Amino-1,6-didesoxynojirimycin

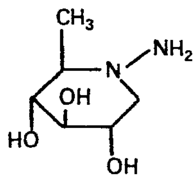

Zu 4,5 g 1,6-Didesoxynojirimycin in 37 ml 2 N HCl tropft man bei 0 °C 6,3 g Natriumnitrit in 30 ml Wasser, rührt 2 Stunden und lässt über Nacht im Kühlschrank stehen. Dann werden unter Eiskühlung 180 ml konzentrierter Ammoniaklösung und anschliessend bei 0 bis 20 °C 16 g Zinkpulver, hinzugegeben. Man rührt 24 Stunden bei Raumtemperatur, kocht 2 Stunden unter Rückfluss, filtriert nach dem Abkühlen vom Niederschlag ab und dampft das Filtrat zur Trockene ein. Der Rückstand wird in wenig 2 N HCl aufgenommen, auf eine Säule mit stark saurem Ionenaustauscher gegeben, mit 2 l Wasser nachgewaschen und mit 0,3 N Ammoniaklösung eluiert. Nach dem Eindampfen des Eluats wird der Rückstand aus Methanol umkristallisiert. Ausbeute 1,3 g, Schmelzpunkt 155 °C.

Die Mutterlaugen werden an Kieselgel (Korngrösse 0,063 bis 0,2 mm) mit Äthanolwasser (10:2) als Laufmittel chromatographiert und ergeben weitere 1,4 g der gewünschten Substanz.

Beispiel 3
N-Benzylidenamino-1-desoxynojirimycin

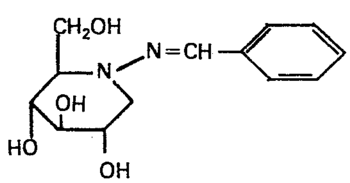

1 g N-Amino-1-desoxynojirimycin wird in 5 ml Methanol und 5 ml Eisessig bei 60 °C gelöst, mit 1,1 ml Benzaldehyd versetzt und bei Raumtemperatur stehen gelassen. Nach kurzer Zeit fällt das Reaktionsprodukt kristallin aus, wird abfiltriert und aus Methanol umkristallisiert. Man erhält 1,4 g vom Schmelzpunkt 203 °C.

Analog dieser Vorschrift werden die Verbindungen der Beispiele 4 bis 16 hergestellt. Bei Verbindungen die nicht direkt kristallin ausfallen, wird die Reaktionsmischung zur Trockene eingedampft und der Rückstand umkristallisiert.

Beispiel 4
N-(2-Hydroxy-3-methoxybenzyliden)-amino-1-desoxynojirimycin

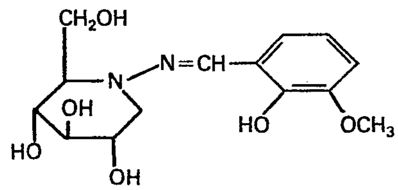

Aus N-Amino-1-desoxynojirimycin und 2-Hydroxy-3-methoxybenzaldehyd, Schmelzpunkt 239 °C.

Beispiel 5
N-(3-Nitrobenzyliden)-amino-1-desoxynojirimycin

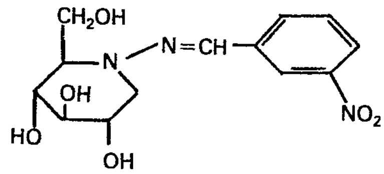

Aus N-Amino-1-desoxynojirimycin und 3-Nitrobenzaldehyd, Schmelzpunkt 205 °C.

Beispiel 6
N-(3-Phenylpropyliden)-amino-1-desoxynojirimycin

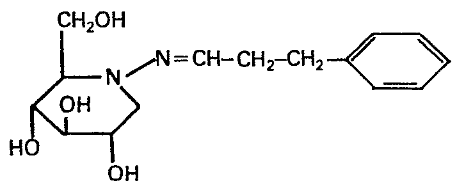

Aus N-Amino-1-desoxynojirimycin und Dihydrozimtaldehyd, Schmelzpunkt 156 °C.

Beispiel 7
N- (4-Methylbenzyliden)-amino-1-desoxynojirimycin

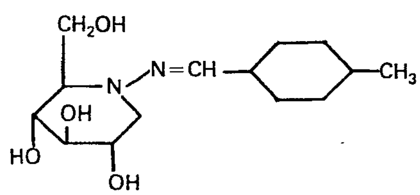

Aus N-Amino-1-desoxynojirimycin und 4-Methylbenzaldehyd, Schmelzpunkt 209 °C.

Beispiel 8
N-(4-Methoxybenzyliden)-amino-1-desoxynojirimicin

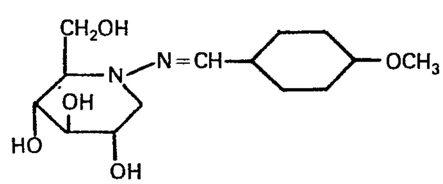

Aus N-Amino-1-desoxynojirimycin und 4-Methoxybenzaldehyd, Schmelzpunkt 209 °C.

Beispiel 9

N-(3-Cyclohexenyl)-methylenamino-1-desoxy-nojirimycin

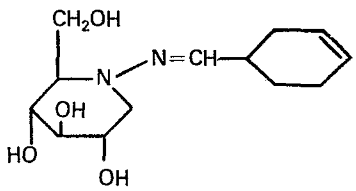

Aus N-Amino-1-desoxynojirimycin und 3-Cyclohexen-1-aldehyd, Schmelzpunkt 143 °C.

Beispiel 10

N-Undecylidenamino- 1-desoxynojirimycin

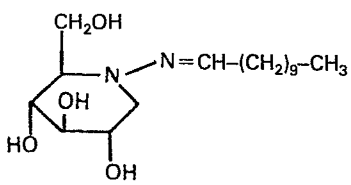

Aus N-Amino-1-desoxynojirimycin und Undecanal, Schmelzpunkt 151 °C.

Beispiel 11

N-Heptylidenamino-1-Desoxynojirimycin

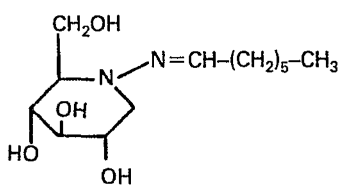

Aus N-Amino-1-desoxynojirimycin und Heptanal, Schmelzpunkt 136 °C.

Beispiel 12

1-Desoxy-N-furfurylidenaminonojirimycin

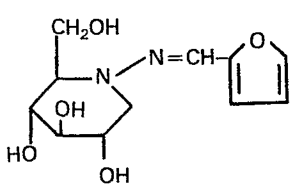

Aus N-Amino-1-desoxynojirimycin und Furfural, Schmelzpunkt 151 bis 152 °C.

Beispiel 13

1-Desoxy-N-thenylidenaminonojirimycin

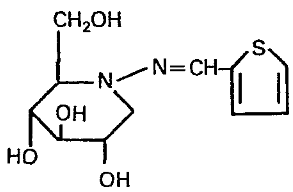

Aus N-Amino-1-desoxynojirimycin und Thiophen-2-aldehyd.

Beispiel 14

1-Desoxy-N-(3-pyridyl)-methylenamino-nojirimycin

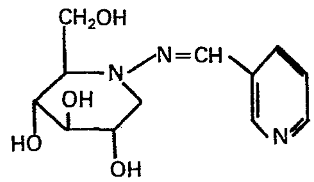

Aus N-Amino-1-desoxynojirimycin und Pyridin-3-aldehyd, Schmelzpunkt 141 °C.

Beispiel 15

N-(4-Chlorbenzyliden)-amino-1-desoxynojirimycin

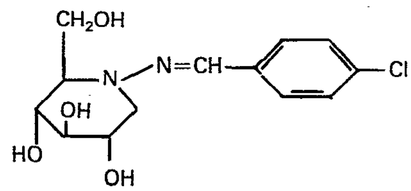

Aus N-Amino-1-desoxynojirimycin und 4-Chlorbenzaldehyd, Schmelzpunkt 206 °C.

Beispiel 16

N-(2-Methylmercaptobenzyliden)-amino-1-desoxynojirimycin

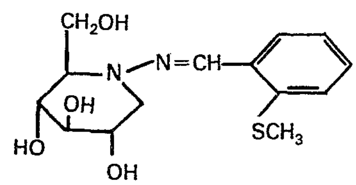

Aus N-Amino-1-desoxynojirimycin und 2-Methylmercaptobenzaldehyd, Schmelzpunkt 178 °C.

Beispiel 17

N-Benzylamino-1-desoxynojirimycin

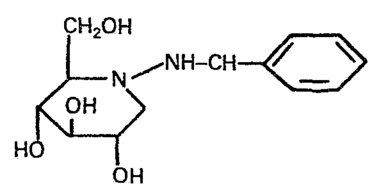

2 g N-Benzylidenamino-1-desoxynojirimycin, 30 ml Methanol, 0,5 g $NaBH_3CN$ und 1 ml Eisessig werden über Nacht bei Raumtemperatur gerührt und anschliessend eine Stunde unter Rückfluss gekocht. Die Reaktionsmischung wird im Vakuum bei 40 °C Badtemperatur eingedampft, in 10 ml 2 N HCl aufgenommen, bis zum Ende der $H_2$-Entwicklung auf dem Wasserbad erwärmt, und auf eine Säule von 20 cm Länge und 2,5 cm Durchmesser, gefüllt mit einem Kationenaustauscher (H+– Form) gegeben. Nach dem Waschen mit 2 l Wasser wird das Reaktionsprodukt mit 0,3 N Ammoniaklösung eluiert, das Eluat eingedampft und der Rückstand aus Methanol umkristallisiert. Man erhält 1,2 g vom Schmelzpunkt 179 °C.

Beispiel 18
N-Acetamido-1-desoxynojirimycin

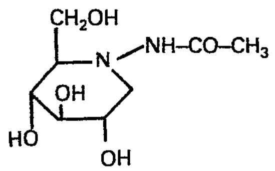

0,5 g N-Amino-1-desoxynojirimycin, 5 ml Wasser, Kationenaustauscher (Amin-Form) und 0,5 ml Acetanhydrid werden 30 Minuten kräftig gerührt. Anschliessend gibt man die Reaktionsmischung auf eine Säule von 10 cm Länge und 1,5 cm Durchmesser, die mit stark basischem Ionenaustauscher (OH-Form) gefüllt ist und eluiert mit Wasser, bis pH 8 erreicht ist. Nach dem Eluieren bleiben 200 mg eines hygroskopischen Schaumes von N-Acetamido-1-desoxynojirimycin zurück, das folgende charakteristische spektroskopische Daten aufweist:

| | | |
|---|---|---|
| Massenspektrum | m/e = | 220 (2%), 202 (15%), 189 (40%), 147 (45%); |
| $^1$H-NMR (100 MHz, $D_2O$) | $\delta$ = | 2,0 (s, $CH_3$-CO) |

Beispiel 19
N-Heptylamino-1-desoxynojirimycin

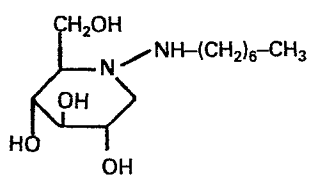

wurde analog Beispiel 17 aus 2 g N-Heptylidenamino-1-desoxynojirimycin erhalten. Ausbeute: 1,85 g Öl.

Beispiel 20
N-Dimethylamino-1-desoxynojirimycin

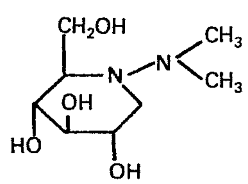

Zu 2 g N-Amino-1-desoxynojirimycin, 5 ml 30%-ige Formalin-Lösung, 15 ml Methanol und 2 g $NaBH_3CN$ tropft man langsam 1,5 ml Eisessig, rührt über Nacht, rotiert den Ansatz ein, löst den Rückstand in 2 N Salzsäure, gibt die Lösung auf einen sauren Ionenaustauscher (Lewatit TSW 40), wässert mit Wasser und eluiert mit 0,3 molarer Lösung von Ammoniak in Methanol/Wasser 10:1. Nach dem Einrotieren des Elauts wird der Rückstand aus Isopropanol/Methanol umkristallisiert. Ausbeute: 1 g, Fp. 177–180 °C.

Beispiel 21
N-Dioctylamino-1-desoxynojirimycin

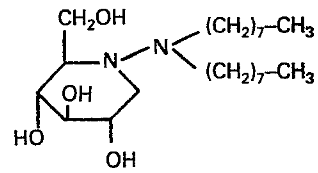

wurde analog Beispiel 20 aus 2 g N-Amino-1-desoxynojirimycin und Octanal statt Formalin hergestellt. Ausbeute: 2,5 g Öl

**Patentansprüche**

1. Verbindungen der allgemeinen Formel (I)

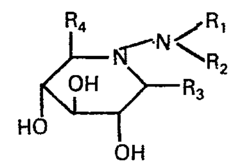

worin

$R_1$ Wasserstoff, $R_5$, Formyl, $COR_5$, $CO_2R_5$, Carbonamid, $CONHR_5$, $CONR_5R_6$, $CSR_5$, $CSNH_2$, $CSNHR_5$, $CSNR_5R_6$, $SO_3H$ oder $SO_2R_5$,

$R_2$ Wasserstoff oder $R_5$ oder $R_1$ und $R_2$ gemeinsam die Gruppierung

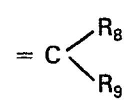

$R_3$ Wasserstoff, Hydroxy, $OR_5$, Mercapto, $SR_5$ Amino, $NHR_5$, $NR_5R_6$, Cyano, Carboxy, $CO_2R_5$, Carbonamido, $CONHR_5$, $CONR_5R_6$, Aminomethyl, $CH_2NHR_5$, $CH_2NR_5R_6$, $CH_2NR_5COR_6$, $CH_2NHSO_2R_5$, $CH_2NR_5SO_2R_6$, Sulfo, Hydroxymethyl, $CH_2OR_5$ oder $CH_2OCOR_5$,

$R_4$ Hydroxymethyl, Hydroxy-($C_1$–$C_6$-Alkyl)-methyl, $C_1$–$C_7$-Alkyl und $C_1$–$C_5$-Alkoxy-methyl,

$R_5$, $R_6$ und $R_7$ unabhängig voneinander einen gegebenenfalls substituierten, geradkettigen, verzweigten oder cyclischen gesättigten oder ungesättigten aliphatischen Kohlenwasserstoffrest oder einen gegebenenfalls substituierten aromatischen oder heterocyclischen Rest, wobei 2 Reste, $R_5$, $R_6$ und $R_7$ auch miteinander verknüpft sein können,

$R_8$ und $R_9$ unabhängig voneinander Wasserstoff oder $R_5$ bedeuten.

2. Verbindungen gemäss Anspruch 1, worin

$R_3$ Wasserstoff, Hydroxy, Sulfo, Cyano, Aminomethyl, $C_1$–$C_6$-Alkylaminomethyl und $C_1$–$C_6$-Alkylcarbonylaminomethyl bedeutet.

3. Verbindungen gemäss Anspruch 1 der allgemeinen Formeln,

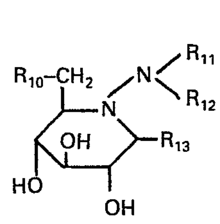
und
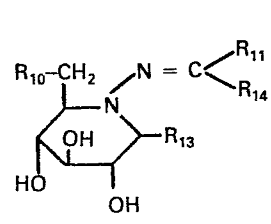

(II) (III)

worin

$R_{10}$ Wasserstoff, Hydroxy oder Methoxy,

$R_{11}$ Wasserstoff, $C_1$–$C_4$-Alkyl, $C_1$–$C_4$-Alkoxy, $C_1$–$C_4$-Alkylsulfonyl, $C_1$–$C_4$-Alkylcarbonyl oder -phenyl, insbesondere Wasserstoff, Methyl, Äthyl, Acetyl, Methylsulfonyl, Propionyl oder Phenyl,

$R_{13}$ Wasserstoff, $C_1$–$C_6$-Alkylcarbonylaminomethyl, Phenylcarbonylaminomethyl, Phenylsulfonylaminomethyl, $C_1$–$C_4$-Alkoxycarbonyl und $C_1$–$C_6$-Alkylaminocarbonyl,

$R_{12}$ Wasserstoff, gegebenenfalls durch Hydroxy, $C_1$–$C_4$-Alkoxy, Carboxy, $C_5$–$C_7$-Cycloalkyl oder Phenylsulfonylamino substituiertes $C_1$–$C_{12}$-Alkyl, $C_2$–$C_4$-Alkenyl, gegebenenfalls im Phenylring durch Halogen, insbesondere Fluor oder Chlor, $C_1$–$C_4$-Alkyl, Hydroxy, Di-$C_1$–$C_4$-Alkylamino, $C_1$–$C_4$-Alkoxy substituiertes Phenyl, Phenyl-$C_1$–$C_4$-alkyl oder Benzoylmethyl, $C_5$–$C_7$-Cycloalkyl, Furylmethyl, Pyridylmethyl, oder Diphenylmethyl und

$R_{14}$ Wasserstoff, gegebenenfalls durch Hydroxy, $C_1$–$C_4$-Alkoxy oder Phenylsulfonylamino substituiertes $C_1$–$C_{12}$-Alkyl, $C_5$–$C_7$-Cycloalkyl, $C_2$–$C_{12}$-Alkenyl, Carboxy, gegebenenfalls durch Halogen, insbesondere Fluor und Chlor, $C_1$–$C_4$-Alkyl, Hydroxy, Nitro, Di-$C_1$–$C_4$-alkylamino, $C_1$–$C_4$-Alkoxy oder -carboxy, substituiertes Phenyl, Phenyl-$C_1$–$C_4$-Alkyl oder -benzoyl, Furyl, Pyridyl oder zusammen mit $R_{11}$ und dem doppelt gebundenen C-Atomen einen Cyclopentan- oder Cyclohexan-Ring bedeuten.

4. Verfahren zur Herstellung der Verbindungen der Formel

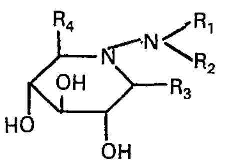

(I)

worin

$R_1$ Wasserstoff, $R_5$, Formyl, $COR_5$, $CO_2R_5$, Carbonamid, $CONHR_5$, $CONR_5R_6$, $CSR_5$, $CSNH_2$, $CSNHR_5$, $CSNR_5R_6$, $SO_3H$ oder $SO_2R_5$,

$R_2$ Wasserstoff oder $R_5$ oder $R_1$ und $R_2$ gemeinsam die Gruppierung

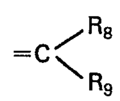

$R_3$ Hydroxy,

$R_4$ Hydroxymethyl, Hydroxy-($C_1$–$C_6$-Alkyl)-methyl, $C_1$–$C_7$-Alkyl und $C_1$–$C_5$-Alkoxy-methyl,

$R_5$, $R_6$ und $R_7$ unabhängig voneinander einen gegebenenfalls substituierten, geradkettigen, verzweigten oder cyclischen gesättigten oder ungesättigten aliphatischen Kohlenwasserstoffrest oder einen gegebenenfalls substituierten aromatischen oder heterocyclischen Rest, wobei 2 Reste $R_5$, $R_6$ und $R_7$ auch miteinander verknüpft sein können,

$R_8$ und $R_9$ unabhängig voneinander Wasserstoff oder $R_5$ bedeuten, dadurch gekennzeichnet, dass man Verbindungen der Formel

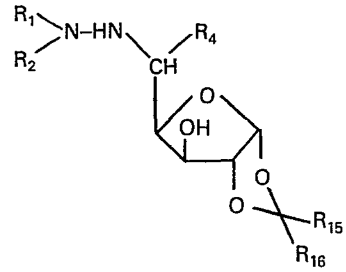

worin

$R_{15}$ und $R_{16}$ unabhängig voneinander $C_1$–$C_4$-Alkyl oder zusammen $C_4$–$C_5$-Alkylen bedeuten und

$R_1$, $R_2$ und $R_4$ die bereits angegebene Bedeutung haben, sauer hydrolysiert.

5. Verfahren zur Herstellung von Verbindungen der allgemeinen Formel

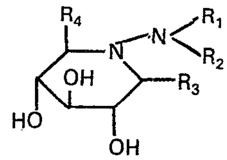

worin

$R_1$ Wasserstoff, $R_5$, Formyl, $COR_5$, $CO_2R_5$, Carbonamid, $CONHR_5$, $CONR_5R_6$, $CSR_5$, $CSNH_2$, $CSNHR_5$, $CSNR_5R_6$, $SO_3H$ oder $SO_2R_5$,

$R_2$ Wasserstoff oder $R_5$ oder $R_1$ und $R_2$ gemeinsam die Gruppierung

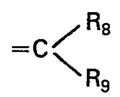

$R_3$ $OR_5$, Mercapto, $SR_5$, Amino, $NHR_5$, $NR_5R_6$, Cyano, Carboxy, $CO_2R_5$, Carbonamido, $CONHR_5$, $CONR_5R_6$, Aminomethyl, $CH_2NHR_5$, $CH_2HR_5R_6$, $CH_2NR_5COR_6$, $CH_2NHSO_2R_5$, $CH_2NR_5SO_2R_6$, Sulfo, Hydroxymethyl, $CH_2OR_5$ doer $CH_2OCOR_5$,

$R_4$ Hydroxymethyl, Hydroxy-($C_1$–$C_6$-Alkyl)-methyl, $C_1$–$C_7$-Alkyl und $C_1$–$C_5$-Alkoxy-methyl,

$R_5$, $R_6$ und $R_7$ unabhängig voneinander einen gegebenenfalls substituierten, geradkettigen, verzweigten oder cyclischen gesättigten oder ungesättigten aliphatischen Kohlenwasserstoffrest oder einen gegebenenfalls substituierten aromatischen oder heterocyclischen Rest, wobei 2 Reste $R_5$, $R_6$ und $R_7$ auch miteinander verknüpft sein können,

$R_8$ und $R_9$ unabhängig voneinander Wasserstoff oder $R_5$ bedeuten, dadurch gekennzeichnet, dass man Verbindungen der allgemeinen Formel

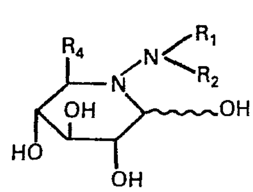

worin

$R_1$, $R_2$ und $R_4$ die bereits genannten Bedeutungen besitzen, mit Nucleophilen umsetzt und die erhaltenen Verbindungen gegebenenfalls verseift

oder reduziert und die Reduktionsprodukte gegebenenfalls alkyliert oder acyliert.

6. Verfahren zur Herstellung von Verbindungen der allgemeinen Formel

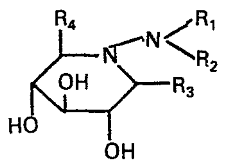

worin

$R_1$ Wasserstoff, $R_5$, Formyl, $COR_5$, $CO_2R_5$, Carbonamid, $CONHR_5$, $CONR_5R_6$, $CSR_5$, $CSNH_2$, $CSNHR_5$, $CSNHR_5R_6$, $SO_3H$ oder $SO_2R_5$,

$R_2$ Wasserstoff oder $R_5$ oder $R_1$ und $R_2$ gemeinsam die Gruppierung

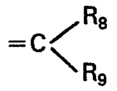

$R_3$ Wasserstoff, $OR_5$, $SR_5$, $NHR_5$, $NR_5R_6$, Cyano, Carboxy, $CO_2R_5$, Carbonamido, $CONHR_5$, $CONR_5R_6$, Aminomethyl, $CH_2NHR_5$, $CH_2NR_5R_6$, $CH_2NR_5COR_6$, $CH_2NHSO_2R_5$, $CH_2NR_5SO_2R_6$, Sulfo, Hydroxymethyl, $CH_2OR_5$ oder $CH_2OCOR_5$,

$R_4$ Hydroxymethyl, Hydroxy-($C_1$–$C_6$-Alkyl)-methyl, $C_1$–$C_7$-Alkyl und $C_1$–$C_5$-Alkoxy-methyl,

$R_5$, $R_6$ und $R_7$ unabhängig voneinander einen gegebenenfalls substituierten, geradkettigen, verzweigten oder cyclischen gesättigten oder ungesättigten aliphatischen Kohlenwasserstoffrest oder einen gegebenenfalls substituierten aromatischen oder heterocyclischen Rest, wobei 2 Reste $R_5$, $R_6$ und $R_7$ auch miteinander verknüpft sein können,

$R_8$ und $R_9$ unabhängig voneinander Wasserstoff oder $R_5$ bedeuten, dadurch gekennzeichnet, dass man Verbindungen der Formel

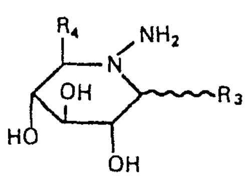

worin

$R_3$ und $R_4$ die genannte Bedeutung aufweisen, alkyliert, aryliert, acyliert oder mit Carbonylverbindungen umsetzt.

7. Verfahren zur Herstellung von Verbindungen der allgemeinen Formel

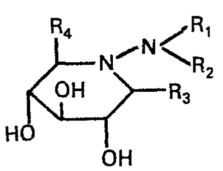

worin

$R_1$ Wasserstoff, $R_5$, Formyl, $COR_5$, $CO_2R_5$, Carbonamid, $CONHR_5$, $CONR_5R_6$, $CSR_5$, $CSNH_2$, $CSNHR_5$, $CSNR_5R_6$, $SO_3H$ oder $SO_2R_5$,

$R_2$ Wasserstoff oder $R_5$ oder $R_1$ und $R_2$ gemeinsam die Gruppierung

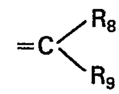

$R_3$ Wasserstoff

$R_4$ Hydroxymethyl, Hydroxy-($C_1$–$C_6$-Alkyl)-methyl, $C_1$–$C_7$-Alkyl und $C_1$–$C_5$-Alkoxy-methyl,

$R_5$, $R_6$ und $R_7$ unabhängig voneinander einen gegebenenfalls substituierten, geradkettigen, verzweigten oder cyclischen gesättigten oder ungesättigten aliphatischen Kohlenwasserstoffrest oder einen gegebenenfalls substituierten aromatischen oder heterocyclischen Rest, wobei 2 Reste $R_5$, $R_6$ und $R_7$ auch miteinander verknüpft sein können,

$R_8$ und $R_9$ unabhängig voneinander Wasserstoff oder $R_5$ bedeuten, dadurch gekennzeichnet, dass man Verbindungen der Formel

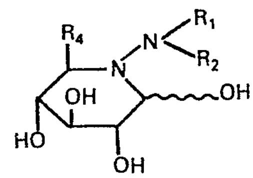

worin

$R_1$, $R_2$ und $R_4$ die genannte Bedeutung besitzen, reduziert.

8. Verfahren zur Herstellung von Verbindungen der Formel

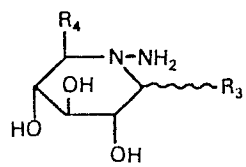

worin

$R_3$ Wasserstoff, Hydroxy, $OR_5$, Mercapto, $SR_5$, Amino, $NHR_5$, $NR_5R_6$, Cyano, Carboxy, $CO_2R_5$, Carbonamido, $CONHR_5$, $CONR_5R_6$, Aminomethyl, $CH_2NHR_5$, $CH_2NR_5R_6$, $CH_2NR_5COR_6$, $CH_2NHSO_2R_5$, $CH_2NR_5SO_2R_6$, Sulfo, Hydroxymethyl, $CH_2OR_5$ oder $CH_2OCOR_5$, und

$R_4$ Hydroxymethyl, Hydroxy-($C_1$–$C_6$-Alkyl)-methyl, $C_1$–$C_7$-Alkyl und $C_1$–$C_5$-Alkoxy-methyl, bedeuten, dadurch gekennzeichnet, dass man Verbindungen der Formel

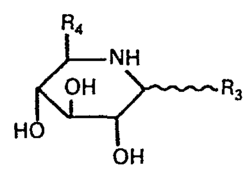

nitrosiert und reduziert.

9. Arzneimittel, gekennzeichnet durch einen Gehalt an einer Verbindung gemäss Anspruch 1 und gegebenenfalls pharmazeutisch geeigneten Zusatzstoffen.

10. Verfahren zur Herstellung eines Arzneimittels, dadurch gekennzeichnet, dass man eine Verbindung gemäss Anspruch 1 gegebenenfalls unter Verwendung pharmazeutisch geeigneter Zusatzstoffe formuliert.

11. Tierfuttermittel, gekennzeichnet durch einen Gehalt an einer Verbindung gemäss Anspruch 1.

12. Verwendung einer Verbindung gemäss Anspruch 1 bei der Tierernährung.

**Revendications**

1. Composés de formule générale (I):

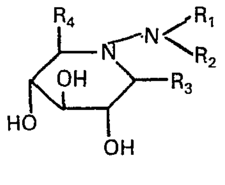

dans laquelle:

$R_1$ est l'hydrogène, un groupe $R_5$, formyle, $COR_5$, $CO_2R_5$, carbonamido, $CONHR_5$, $CONR_5R_6$, $CSR_5$, $CSNH_2$, $CSNHR_5$, $CSNR_5R_6$, $SO_3H$ ou $SO_2R_5$,

$R_2$ est l'hydrogène ou $R_5$ ou $R_1$ et $R_2$ forment en commun le groupement:

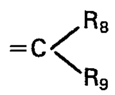

$R_3$ représente l'hydrogène, un groupe hydroxy, $OR_5$, mercapto, $SR_5$, amino, $NRH_5$, $NR_5R_6$, cyano, carboxy, $CO_2R_5$, carbonamido, $CONHR_5$, $CONR_5R_6$, aminométhyle, $CH_2NHR_5$, $CH_2NR_5R_6$, $CH_2NHR_5$, $CH_2NR_5COR_6$, $CH_2NHSO_2R_5$, $CH_2NR_5SO_2R_6$, sulfo, hydroxyméthyle, $CH_2OR_5$ ou $CH_2OCOR_5$,

$R_4$ est un groupe hydroxyméthyle, hydroxy-(alkyle en $C_1$ à $C_6$)-méthyle, alkyle en $C_1$ à $C_7$ et (alkoxy en $C_1$ à $C_5$)-méthyle,

$R_5$, $R_6$ et $R_7$ forment, indépendamment l'un de l'autre, un reste hydrocarboné aliphatique saturé ou non saturé à chaîne droite, ramifié ou cyclique éventuellement substitué ou un reste aromatique ou hétérocyclique éventuellement substitué, deux restes $R_5$, $R_6$ et $R_7$ pouvant aussi être liés ensemble,

$R_8$ et $R_9$ désignent, indépendamment l'un de l'autre, de l'hydrogène ou $R_5$.

2. Composés suivant la revendication 1, dans lesquels $R_3$ représente l'hydrogène ou un groupe hydroxy, sulfo, cyano, aminométhyle, (alkyle en $C_1$ à $C_6$-amino-méthyle ou (alkyle en $C_1$ à $C_6$)-carbonylaminométhyle.

3. Composés suivant la revendication 1, de formules générales:

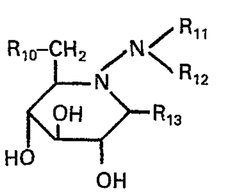

und

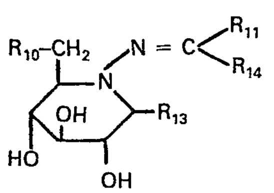

(II) (III)

où

$R_{10}$ est l'hydrogène, le groupe hydroxy ou le groupe méthoxy,

$R_{11}$ est l'hydrogène ou un groupe alkyle en $C_1$ à $C_4$, alkoxy en $C_1$ à $C_4$, alkylsulfonyle en $C_1$ à $C_4$, (alkyle en $C_1$ à $C_4$)-carbonyle ou (alkyle en $C_1$ à $C_4$)-phényle, notamment l'hydrogène ou le groupe méthyle, éthyle, acétyle, méthylsulfonyle, propionyle ou phényle,

$R_{13}$ désigne l'hydrogène ou un groupe (alkyle en $C_1$ à $C_6$)-carbonylaminométhyle, phénylcarbonyl-amino-méthyle, phénylsulfonylaminométhyle, (alkoxy en $C_1$ à $C_4$)-carbonyle et (alkyle en $C_1$ à $C_6$)-amino-carbonyle,

$R_{12}$ est l'hydrogène, un groupe alkyle en $C_1$ à $C_{12}$ éventuellement substitué par un radical hydroxy, alkoxy en $C_1$ à $C_4$, carboxy, cycloalkyle en $C_5$ à $C_7$ ou phénylsulfonylamino, un groupe alcényle en $C_2$ à $C_4$, un groupe phényle, phényl-(alkyle en $C_1$ à $C_4$) ou Benzoylméthyle éventuellement substitué dans le noyau phényle par un halogène, notamment le fluor ou le chlore, un radical alkyle en $C_1$ à $C_4$, hydroxy, di-(alkyle en $C_1$ à $C_4$)-amino ou alkoxy en $C_1$ à $C_4$, un groupe cycloalkyle en $C_4$ à $C_7$, furylméthyle, pyridylméthyle ou diphényl-méthyle, et

$R_{14}$ est l'hydrogène, un groupe alkyle en $C_1$ à $C_{12}$ éventuellement substitué par un radical hydroxy, alkoxy en $C_1$ à $C_4$ ou phénylsulfonylamino, un groupe cycloalkyle en $C_5$ à $C_7$, alcényle en $C_2$ à $C_{12}$, carboxy, un groupe phényle, phényl-(alkyle en $C_1$ à $C_4$) ou – benzoyle éventuellement substitué par un halogène, notamment le fluor et le chlore ou un radical alkyle en $C_1$ à $C_4$, hydroxy, nitro, di-(alkyle en $C_1$ à $C_4$)-amino, alkoxy en $C_1$ à $C_4$ ou carboxy en $C_1$ à $C_4$, un groupe furyle ou pyridyle, ou forme conjointement avec $R_{11}$ et l'atome de carbone portant la double liaison, un noyau cyclopentane ou cyclohexane.

4. Procédé de production des composés de formule:

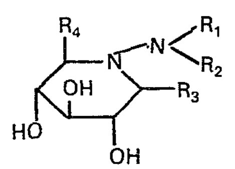

(I)

dans laquelle:

$R_1$ désigne l'hydrogène, un groupe $R_5$, formyle, $COR_5$, $CO_2R_5$, carbonamido, $CONHR_5$, $CONR_5R_6$, $CSR_6$, $CSNH_2$, $CSNHR_5$, $CSNR_5R_6$, $SO_3H$ ou $SO_2R_5$,

$R_2$ est l'hydrogène ou $R_5$, ou $R_1$ et $R_2$ forment ensemble le groupement:

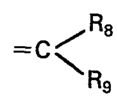

$R_3$ est un groupe hydroxy,

$R_4$ est un groupe hydroxyméthyle, hydroxy-(alkyle en $C_1$ à $C_6$)-méthyle, alkyle en $C_1$ à $C_7$ et (alkoxy en $C_1$ à $C_5$)-méthyle,

$R_5$, $R_6$ et $R_7$ forment, indépendamment l'un de l'autre, un reste d'hydrocarbure aliphatique saturé

ou non saturé à chîne droite, ramifié ou cyclique éventuellement substitué ou un reste aromatique ou hétérocyclique éventuellement substitué, deux restes $R_5$, $R_6$ et $R_7$ pouvant aussi être liés ensemble,

$R_8$ et $R_9$ désignent, indépendamment l'un de l'autre, l'hydrogène ou le groupe $R_5$, caractérisé en ce qu'on effectue l'hydrolyse acide de composés de formule:

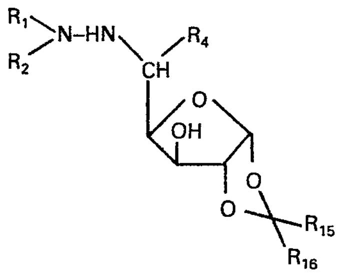

dans laquelle:

$R_{15}$ et $R_{16}$ désignent, indépendamment l'un de l'autre, un groupe alkyle en $C_1$ à $C_4$ ou forment ensemble un groupe alkylène en $C_4$ ou $C_5$, et

$R_1$, $R_2$ et $R_4$ ont la définition déjà indiquée.

5. Procédé de production de composés de formule générale:

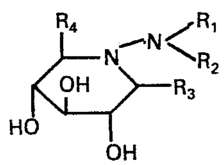

dans laquelle:

$R_1$ désigne l'hydrogène, un groupe $R_5$, formyle, $COR_5$, $CO_2R_5$, carbonamido, $CONHR_5$, $CONR_5R_6$, $CSR_5$, $CSNH_2$, $CSNHR_5$, $CSNR_5R_6$, $SO_3H$ ou $SO_2R_5$,

$R_2$ est l'hydrogène ou un groupe $R_5$ ou $R_1$ et $R_2$ forment ensemble le groupement:

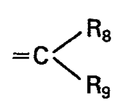

$R_3$ est un groupe $OR_5$, mercapto, $SR_5$, amino, $NRH_5$, $NR_5R_6$, cyano, carboxy, $CO_2R_5$, carbonamido, $CONHR_5$, $CONR_5R_6$, aminométhyle, $CH_2NHR_5$, $CH_2NR_5R_6$, $CH_2NHR_5$, $CH_2NR_5COR_6$, $CH_2NHSO_2R_5$, $CH_2NR_5SO_2R_6$, sulfo, hydroxyméthyle, $CH_2OR_5$ ou $CH_2OCOR_5$,

$R_4$ est le groupe hydroxyméthyle, hydroxy-(alkyle en $C_1$ à $C_6$)-méthyle, alkyle en $C_1$ à $C_7$ et (alkoxy en $C_1$ à $C_5$)-méthyle,

$R_5$, $R_6$ et $R_7$ désignent, indépendamment l'un de l'autre, un reste d'hydrocarbure aliphatique saturé ou non saturé à chaîne droite, ramifié ou cyclique éventuellement substitué ou un reste aromatique ou hétérocyclique éventuellement substitué, deux restes $R_5$, $R_6$ et $R_7$ pouvant également être liés ensemble,

$R_8$ et $R_9$ désignent, indépendamment l'un de l'autre, de l'hydrogène ou un groupe $R_5$, caractérisé en ce qu'on fait réagir des composés de formule générale:

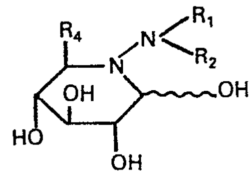

dans laquelle:

$R_1$, $R_2$ et $R_4$ ont les définitions déjà connues, avec des nucléophiles et on saponifie ou on réduit éventuellement les composés obtenus et on alkyle ou acyle éventuellement les produits de réduction.

6. Procédé de production de composés de formule générale:

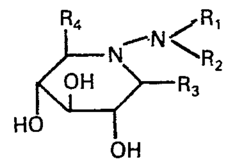

dans laquelle:

$R_1$ est l'hydrogène ou un groupe $R_5$, formyle, $COR_5$, $CO_2R_5$, carbonamido, $CONHR_5$, $CONR_5R_6$, $CSR_5$, $CSNH_2$, $CSNHR_5$, $CSNR_5R_6$, $SO_3H$ ou $SO_2R_5$,

$R_2$ est l'hydrogène ou $R_5$, ou $R_1$ et $R_2$ forment ensemble le groupement:

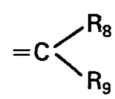

$R_3$ est l'hydrogène ou un groupe $OR_5$, $SR_5$, $NRH_5$, $NR_5R_6$, cyano, carboxy, $CO_2R_5$, carbonamido, $CONHR_5$, $CONR_5R_6$, aminométhyle, $CH_2NHR_5$, $CH_2NR_5R_6$, $CH_2NHR_5$, $CH_2NR_5COR_6$, $CH_2NHSO_2R_5$, $CH_2NR_5SO_2R_6$, sulfo, hydroxyméthyle, $CH_2OR_5$ ou $CH_2OCOR_5$,

$R_4$ est un groupe hydroxyméthyle, hydroxy-(alkyle en $C_1$ à $C_6$)-méthyle, alkyle en $C_1$ à $C_7$ et (alkoxy en $C_1$ à $C_5$)-méthyle,

$R_5$, $R_6$ et $R_7$ désignent, indépendamment l'un de l'autre, un reste d'hydrocarbure aliphatique saturé ou non saturé à chaîne droite, ramifié ou cyclique éventuellement substitué ou un reste aromatique ou hétérocyclique éventuellement substitué, deux restes $R_5$, $R_6$ et $R_7$ pouvant également être liés ensemble,

$R_8$ et $R_9$ désignent, indépendamment l'un de l'autre, l'hydrogène ou un groupe $R_5$, caractérisé en ce qu'on alkyle, on aryle, on acyle ou on fait réagir avec des composés carbonyliques des composés de formule:

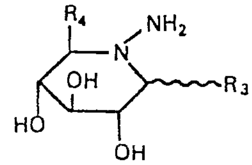

dans laquelle:

$R_3$ et $R_4$ ont la définition mentionnée.

7. Procédé de production de composés de formule générale:

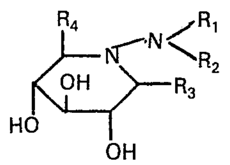

dans laquelle:

$R_1$ désigne l'hydrogène ou un groupe $R_5$, formyle, $COR_5$, $CO_2R_5$, carbonamido, $CONHR_5$, $CONR_5R_6$, $CSR_5$, $CSNH_2$, $CSNHR_5$, $CSNR_5R_6$, $SO_3H$ ou $SO_2R_5$,

$R_2$ est l'hydrogène ou un groupe $R_5$ ou $R_1$ et $R_2$ forment ensemble le groupement:

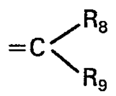

$R_3$ est l'hydrogène,

$R_4$ est un groupe hydroxyméthyle, hydroxy-(alkyle en $C_1$ à $C_6$)-méthyle, alkyle en $C_1$ à $C_7$ et (alkoxy en $C_1$ à $C_5$)-méthyle,

$R_5$, $R_6$ et $R_7$ désignent, indépendamment l'un de l'autre, un reste d'hydrocarbure aliphatique saturé ou non saturé à chaîne droite, ramifié ou cyclique éventuellement substitué ou un reste aromatique ou hétérocyclique éventuellement substitué, deux restes $R_5$, $R_6$ et $R_7$ pouvant aussi être liés ensemble,

$R_8$ et $R_9$ désignent, indépendamment l'un de l'autre, l'hydrogène ou un groupe $R_5$, caractérisé en ce qu'on réduit des composés de formule:

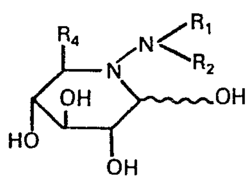

dans laquelle:

$R_1$, $R_2$ et $R_4$ ont la définition mentionnée.

8. Procédé de production de composés de formule:

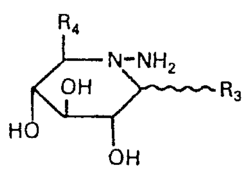

dans laquelle:

$R_3$ désigne l'hydrogène ou un groupe hydroxy, $OR_5$, mercapto, $SR_5$, amino, $NRH_5$, $NR_5R_6$, cyano, carboxy, $CO_2R_5$, carbonamido, $CONHR_5$, $CONR_5R_6$, aminométhyle, $CH_2NHR_5$, $CH_2NR_5R_6$, $CH_2NHR_5$, $CH_2NR_5COR_6$, $CH_2NHSO_2R_5$, $CH_2NR_5SO_2R_6$, sulfo, hydroxyméthyle, $CH_2OR_5$ ou $CH_2OCOR_5$, et

$R_4$ est un groupe hydroxyméthyle, hydroxy-(alkyle en $C_1$ à $C_6$)-méthyle, alkyle en $C_1$ à $C_7$ et (alkoxy en $C_1$ à $C_5$)-méthyle, caractérisé en ce qu'on nitrose et réduit des composés de formule:

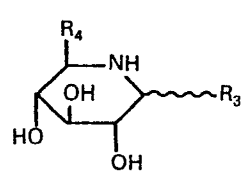

9. Médicament, caractérise par une teneur en un composé suivant la revendication 1 et, le cas échéant, en additifs pharmaceutiquement acceptables.

10. Procédé de préparation d'un médicament, caractérisé en ce qu'on formule un composé suivant la revendication 1 en utilisant éventuellement des additifs pharmaceutiquement acceptables.

11. Aliment pour le bétail, caractérisé par une teneur en un composé suivant la revendication 1.

12. Utilisation d'un composé suivant la revendication 1 dans l'alimentation des animaux.

**Claims**

1. Compounds of the general formula (I)

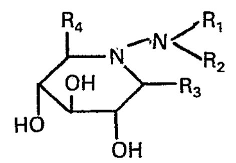

wherein

$R_1$ denotes hydrogen, $R_5$, formyl, $COR_5$, $CO_2R_5$, carboxamide, $CONHR_5$, $CONR_5R_6$, $CSR_5$, $CSNH_2$, $CSNHR_5$, $CSNR_5R_6$, $SO_3H$ or $SO_2R_5$,

$R_2$ denotes hydrogen or $R_5$ or $R_1$ and $R_2$ together denote the grouping

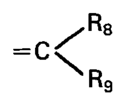

$R_3$ denotes hydrogen, hydroxyl, $OR_5$, mercapto, $SR_5$, amino, $NRH_5$, $NR_5R_6$, cyano, carboxyl, $CO_2R_5$, carboxamido, $CONHR_5$, $CONR_5R_6$, aminomethyl, $CH_2NHR_5$, $CH_2NR_5R_6$, $CH_2NHR_5$, $CH_2NR_5COR_6$, $CH_2NHSO_2R_5$, $CH_2NR_5SO_2R_6$, sulpho, hydroxymethyl, $CH_2OR_5$ or $CH_2OCOR_5$,

$R_4$ denotes hydroxymethyl, hydroxy-($C_1$–$C_6$-alkyl)-methyl, $C_1$–$C_7$-alkyl and $C_1$–$C_5$-alkoxymethyl,

$R_5$, $R_6$ and $R_7$ independently of one another denote an optionally substituted, straight-chain, branched or cyclic saturated or unsaturated aliphatic hydrocarbon radical or an optionally substituted aromatic or heterocyclic radical, it also being possible for 2 radicals $R_5$, $R_6$ and $R_7$ to be linked to one another,

$R_8$ and $R_9$ independently of each other denote hydrogen or $R_5$.

2. Compounds according to Claim 1, wherein $R_3$ denotes hydrogen, hydroxy, sulpho, cyano, aminomethyl, $C_1$–$C_6$-alkylamino-methyl and $C_1$–$C_6$-alkylcarbonylaminomethyl.

3. Compounds according to Claim 1 of the general formula

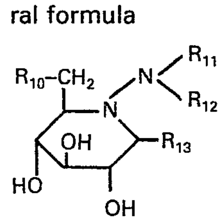

(II)

und

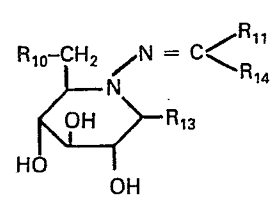

(III)

wherein

$R_{10}$ denotes hydrogen, hydroxyl or methoxy,

$R_{11}$ denotes hydrogen, $C_1$–$C_4$-alkyl, $C_1$–$C_4$-alkoxy, $C_1$–$C_4$-alkylsulphonyl, $C_1$–$C_4$-alkylcarbonyl or phenyl, in particular hydrogen, methyl, ethyl, acetyl, methyl-sulphonyl, propionyl or phenyl,

$R_{13}$ denotes hydrogen, $C_1$–$C_6$-alkylcarbonylamiomethyl, phenylcarbonylaminomethyl, phenylsulphonylaminomethyl, $C_1$–$C_4$-alkoxycarbonyl and $C_1$–$C_6$-alkylaminocarbonyl,

$R_{12}$ denotes hydrogen, $C_1$–$C_{12}$-alkyl which is optionally substituted by hydroxyl, $C_1$–$C_4$-alkoxy, carboxyl, $C_5$–$C_7$-cycloalkyl or phenylsulphonylamino, $C_2$–$C_4$-alkenyl, phenyl, phenyl-$C_1$–$C_4$-alkyl or benzoylmethyl which is optionally substituted in the phenyl ring by halogen, in particular fluorine or chlorine, $C_1$–$C_4$-alkyl, hydroxyl, di-$C_1$–$C_4$-alkylamino or $C_1$–$C_4$-alkoxy, $C_5$–$C_7$-cycloalkyl, furylmethyl, pyridylmethyl or diphenylmethyl and

$R_{14}$ denotes hydrogen, $C_1$–$C_{12}$-alkyl which is optionally substituted by hydroxyl, $C_1$–$C_4$-alkoxy or phenylsulphonylamino, $C_5$–$C_7$-cycloalkyl, $C_2$–$C_{12}$-alkenyl, carboxyl, phenyl, phenyl-$C_1$–$C_4$-alkyl or benzoyl which is optionally substituted by halogen, in particular fluorine and chlorine, $C_1$–$C_4$-alkyl, hydroxyl, nitro, di-$C_1$–$C_4$-alkyl-amino, $C_1$–$C_4$-alkoxy or carboxyl, furyl or pyridyl, or together with $R_{11}$ and the double-bonded carbon atoms, completes a cyclopentane or cyclohexane ring.

4. Process for the production of the compounds of the formula

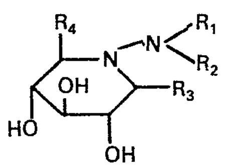

wherein

$R_1$ denotes hydrogen, $R_5$, formyl, $COR_5$, $CO_2R_5$, carboxamide, $CONHR_5$, $CONR_5R_6$, $CSR_5$, $CSNH_2$, $HR_5$, $CSNR_5R_6$, $SO_3H$ or CSN $SO_2R_5$,

$R_2$ denotes hydrogen or $R_5$ or $R_1$ and $R_2$ together denote the grouping

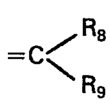

$R_3$ denotes hydroxyl,

$R_4$ denotes hydroxymethyl, hydroxy-($C_1$–$C_6$-alkyl)-methyl, $C_1$–$C_7$-alkyl and $C_1$–$C_5$-alkoxymethyl,

$R_5$, $R_6$ and $R_7$ independently of one another denote an optionally substituted or unsaturated aliphatic hydrocarbon radical or an optionally substituted aromatic or heterocyclic radical, it also being possible for 2 radicals $R_5$, $R_6$ and $R_7$ to be linked to one another,

$R_8$ and $R_9$ independently of each other denote hydrogen or $R_5$, characterised in that compounds of the formula

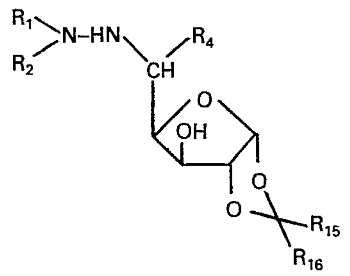

wherein

$R_{15}$ and $R_{16}$ independently of one another denote $C_1$–$C_4$-alkyl or together denote $C_4$–$C_5$-alkylene and

$R_1$, $R_2$ and $R_4$ have the meaning already indicated, are subjected to acid hydrolysis.

5. Process for the production of compounds of the general formula

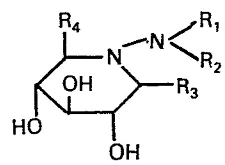

wherein

$R_1$ denotes hydrogen, $R_5$, formyl, $COR_5$, $CO_2R_5$, carboxamide, $CONHR_5$, $CONR_5R_6$, $CSR_5$, $CSNH_2$, $CSNHR_5$, $CSNR_5R_6$, $SO_3H$ or $SO_2R_5$,

$R_2$ denotes hydrogen or $R_5$ or $R_1$ and $R_2$ together denote the grouping

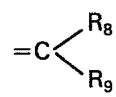

$R_3$ denotes $OR_5$, mercapto, $SR_5$, amino, $NRH_5$, $NR_5R_6$, cyano, carboxyl, $CO_2R_5$, carboxamido, $CONHR_5$, $CONR_5R_6$, aminomethyl, $CH_2NHR_5$, $CH_2NR_5R_6$, $CH_2NHR_5$, $CH_2NR_5COR_6$, $CH_2NHSO_2R_5$, $CH_2NR_5SO_2R_6$, sulpho, hydroxymethyl, $CH_2OR_5$ or $CH_2OCOR_5$,

$R_4$ denotes hydroxymethyl, hydroxy-($C_1$–$C_6$-alkyl)-methyl, $C_1$–$C_7$-alkyl and $C_1$–$C_5$-alkoxymethyl,

$R_5$, $R_6$ and $R_7$ independently of one another denote an optionally substituted, straight-chain, branched or cyclic saturated or unsaturated aliphatic hydrocarbon radical or an optionally substituted aromatic or heterocyclic radical, it also being possible for 2 radicals $R_5$, $R_6$ and $R_7$ to be linked to one another,

$R_8$ and $R_9$ independently of each other denote hydrogen or $R_5$,

characterised in that compounds of the general formula

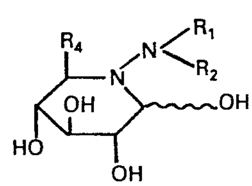

wherein

$R_1$, $R_2$ and $R_4$ have the meanings already known, are reacted with nucelophiles and, if appropriate,

the resulting compounds are saponified or reduced and, if appropriate, the reduction products are alkylated or acylated.

6. Process for the production of compounds of the general formula.

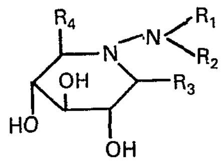

wherein

$R_1$ denotes hydrogen, $R_5$, formyl, $COR_5$, $CO_2R_5$, carboxamide, $CONHR_5$, $CONR_5R_6$, $CSR_5$, $CSNH_2$, $CSNHR_5$, $CSNR_5R_6$, $SO_3H$ or $SO_2R_5$,

$R_2$ denotes hydrogen or $R_5$ or $R_1$ and $R_2$ together denote the grouping

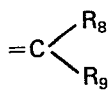

$R_3$ denotes hydrogen, $OR_5$, $SR_5$, $NRH_5$, $NR_5R_6$, cyano, carboxyl- $CO_2R_5$, carboxamido, $CONHR_5$, $CONHR_5R_6$, aminomethyl, $CH_2NHR_5$, $CH_2NR_5R_6$, $CH_2NHR_5$, $CH_2NR_5COR_6$, $CH_2NHSO_2R_5$, $CH_2NR_5SO_2R_6$, sulpho, hydroxymethyl, $CH_2OR_5$ or $CH_2OCOR_5$,

$R_4$ denotes hydroxymethyl, hydroxy-($C_1$–$C_6$-alkyl)-methyl, $C_1$–$C_7$-alkyl and $C_1$–$C_5$-alkoxymethyl,

$R_5$, $R_6$ and $R_7$ independently of one another denote an optionally substituted, straight-chain, branched or cyclic saturated or unsaturated aliphatic hydrocarbon radical or an optionally substituted aromatic or heterocyclic radical, it also being possible for 2 radicals $R_5$, $R_6$ and $R_7$ to be linked to one another,

$R_8$ and $R_9$ independently of each other denote hydrogen or $R_5$, characterised in that compounds of the formula

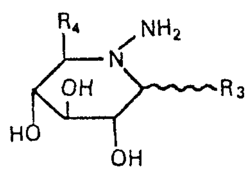

wherein

$R_3$ and $R_4$ have the indicated meaning, are alkylated, arylated, acylated or reacted with carbonyl compounds.

7. Process for the production of compounds of the general formula

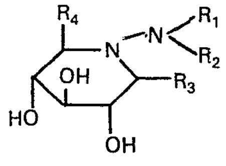

wherein

$R_1$ denotes hydrogen, $R_5$, formyl, $COR_5$, $CO_2R_5$, carboxamide, $CONHR_5$, $CONR_5R_6$, $CSR_5$, $CSNH_2$, $CSNHR_5$, $CSNR_5R_6$, $SO_3H$ or $SO_2R_5$,

$R_2$ denotes hydrogen or $R_5$ or $R_1$ and $R_2$ together denote the grouping

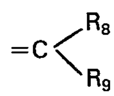

$R_3$ denotes hydrogen,

$R_4$ denotes hydroxymethyl, hydroxy-($C_1$–$C_6$-alkyl)-methyl, $C_1$–$C_7$-alkyl and $C_5$–$C_7$-akoxymethyl,

$R_5$, $R_6$ and $R_7$ independently of one another denote an optionally substituted, straight-chain, branched or cyclic saturated or unsaturated aliphatic hydrocarbon radical or an optionally substituted aromatic or heterocyclic radical, it also being possible for 2 radicals $R_5$, $R_6$ and $R_7$ to be linked to one another,

$R_8$ and $R_9$ independently of each other denote hydrogen or $R_5$, characterised in that compounds of the formula

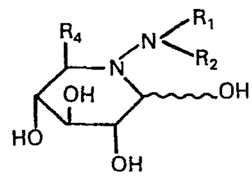

wherein

$R_1$, $R_2$ and $R_4$ have the indicated meaning, are reduced.

8. Process for the production of compounds of the formula

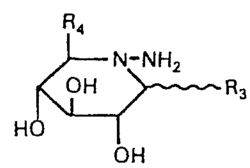

wherein

$R_3$ denotes hydrogen, hydroxy, $OR_5$, mercapto, $SR_5$, amino, $NRH_5$, $NR_5R_6$, cyano, carboxyl, $CO_2R_5$, carboxamido, $CONHR_5$, $CONHR_5R_6$, aminomethyl, $CH_2NHR_5$, $CH_2NR_5R_6$, $CH_2NHR_5$, $CH_2NR_5COR_6$, $CH_2NHSO_2R_5$, $CH_2NR_5SO_2R_6$, sulpho, hydroxymethyl, $CH_2OR_5$ or $CH_2OCOR_5$, and

$R_4$ denotes hydroxymethyl, hydroxy-($C_1$–$C_6$-alkyl)-methyl, $C_1$–$C_7$-alkyl and $C_1$–$C_5$-alkoxymethyl, characterised in that compounds of the formula

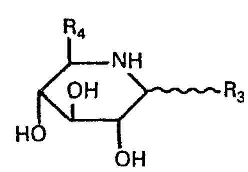

are nitrosated and the nitrosation products are reduced.

9. Medicaments, characterised in that they contain a compound according to Claim 1 and , if appropriate, pharmaceutically suitable additives.

10. Process for the production of a medicament, characterised in that a compound according to Claim 1 is made into formulations, using pharmaceutically suitable additives if appropriate.

11. Animal feddstuff, characterised in that it contains a compound according to Claim 1.

12. Use of a compound according to Claim 1 for the nutrition of animals.